(19) Europäisches Patentamt
European Patent Office
Office européen des brevets

(11) EP 2 354 221 A2

# (12) EUROPEAN PATENT APPLICATION

(43) Date of publication:
**10.08.2011 Bulletin 2011/32**

(51) Int Cl.:
***C12N 7/00*** *(2006.01)* ***C12N 7/04*** *(2006.01)*
***A61K 39/12*** *(2006.01)*

(21) Application number: **10163605.8**

(22) Date of filing: **21.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **04.02.2010 KR 20100010656**

(71) Applicant: **Green Cross Veterinary Products Co., Ltd.**
**Chungcheongnam-Do 340-861 (KR)**

(72) Inventors:
- **Song, Dae Sub**
  **Suwon-si**
  **Gyeonggi-do 440-330 (KR)**
- **Kang, Bo Kyu**
  **Yongsin-si**
  **Gyeonggi-do 446-762 (KR)**
- **Kim, Hye Kwon**
  **Incheon-city 405-221 (KR)**
- **Park, Bong Kyun**
  **Suwon-si**
  **Gyeonggi-Do 441-440 (KR)**

(74) Representative: **Augarde, Eric et al**
**BREVALEX**
**56 Boulevard de l'Embouchure,**
**Bât. B**
**B.P. 27519**
**31075 Toulouse Cedex 2 (FR)**

## (54) Porcine circovirus type 2 and use thereof

(57) Porcine circovirus type 2 (PCV2) having high replication ability in host cells and use thereof.

EP 2 354 221 A2

Printed by Jouve, 75001 PARIS (FR)

## Description

CROSS-REFERENCE TO RELATED PATENT APPLICATION

This application claims the benefit of Korean Patent Application No. 10-2010-0010656, filed on February 4, 2010, in the Korean Intellectual Property Office, the disclosure of which is incorporated herein in its entirety by reference.

BACKGROUND OF THE INVENTION

1. Field of the Invention

The present invention relates to a porcine circovirus type 2 and use thereof.

2. Description of the Related Art

Porcine circovirus type 2 (PCV2) is a small, icosahedral, non-enveloped DNA virus having a diameter in the range of 17 to 22 nm with a single-stranded circular genome. PCV2 and porcine circovirus type 1 (PCV1) have a degree of sequence identity of about 80%. While PCV1 is non-pathogenic, PCV2-infected pigs have symptoms such as postweaning multisystemic wasting syndrome (PMWS), porcine respiratory disease complex (PRDC), PCV2-caused enteritis, PCV2-caused reproductive diseases, porcine dermatitis nephritis syndrome (PDNS), atrophy, respiratory symptoms, diarrhea, jaundice, and gastric ulcer. The PCV2 is a member of the family of Circoviridae. The PCV2 genome does not encode for a viral DNA-polymerase, making it dependent on cellular enzymes to complete its infectious cycle.

When PCV2 is inoculated in pigs, a high variation in virus replication is observed. It has been observed that PCV2 is able to replicate better in a host when simultaneously inoculated with other viruses such as porcine reproductive and respiratory syndrome virus (PRRSV) or porcine parvovirus (Allan et al (2000) Arch Virol. 145, 2421-2429, Allan et al (2000) J Vet Med B. 47, 81-94).

The titer of PCV2 present in a cell culture infected by PCV2 is generally in the range of 3.4 to 4.5 $log_{10}$ $TCID_{50}$/ml. U.S. Patent No. 7,566,562 discloses a method for in vitro cultivation of a porcine circovirus, the method including: inoculating cells of a continuous animal cell line in a culture medium with porcine circovirus; cultivating the continuous animal cell line in the medium in the presence of one or more inhibitors of endosomal-lysosomal system acidification; and isolating the porcine circovirus from the medium and/or the infected continuous animal cell line.

However, there is still a need to develop PCV2 having high replication ability in host cells. In particular, there is still a need to develop PCV2 having high replication ability without using a virus replication enhancer such as at least one inhibitor of endosomal-lysosomal system acidification.

SUMMARY OF THE INVENTION

The present invention provides a porcine circovirus type 2 (PCV2) having high replication ability in a host cell.

The present invention also provides a method of attenuating a PCV2 having high replication ability in a host cell.

The present invention also provides an immunogenic composition including an attenuated PCV2 having high replication ability in a host cell.

The present invention also provides a method of preventing or treating PCV2 infection-associated diseases in animals by using the immunogenic composition.

According to an aspect of the present invention, there is provided a composition for treating or preventing circovirus infection in animals, the composition comprising at least on attenuated virus selected from the group consisting of attenuated circovirus PCV2 GC0504 and PCV2 GC0505.

According to another aspect of the present invention, there is provided a method of producing attenuated circovirus, the method comprising reacting at least one virus selected from the group consisting of circovirus PCV2 GC0504 and PCV2 GC0505 with formalin to prepare attenuated circovirus.

According to another aspect of the present invention, there is provided a method of treating or preventing circovirus infection in animals, the method comprising administering the composition according to any one of claims 1 in an effective amount into the animals.

According to another aspect of the present invention, there is provided a Circovirus PCV2 GC0504 comprising a genome having a nucleotide sequence of SEQ ID NO: 1.

According to another aspect of the present invention, there is provided a Circovirus PCV2 GC0505 comprising a genome having a nucleotide sequence of SEQ ID NO: 4.

## BRIEF DESCRIPTION OF THE DRAWINGS

The above and other features and advantages of the present invention will become more apparent by describing in detail exemplary embodiments thereof with reference to the attached drawings in which:

FIG. 1 is a graph illustrating results of real time-polymerase chain reaction (PCR) of PCV2 having various concentrations;
FIG. 2 is a graph illustrating PCV2 elution via nasal cavity after PCV2 challenge-inoculation into young pigs;
FIG. 3 is a graph illustrating PCV2 elution via nasal cavity after challenge-inoculation of PCV2 and porcine reproductive and respiratory syndrome virus (PRRSV) into young pigs;
FIG. 4 is a graph illustrating PCV2 elution via excrement after PCV2 challenge-inoculation into young pigs; and
FIG. 5 is a graph illustrating PCV2 elution via excrement after challenge-inoculation of PCV2 and PRRSV into young pigs.

## DETAILED DESCRIPTION OF THE INVENTION

Hereinafter, the present invention will now be described more fully with reference to the accompanying drawings, in which exemplary embodiments of the invention are shown.

One or more embodiments of the present invention provide a composition for treating or preventing circovirus infection in animals, the composition including at least one attenuated virus selected from the group consisting of attenuated circoviruses PCV2 GC0504 and PCV2 GC0505.

PCV2 GC0504 may include a genome having a nucleotide sequence of SEQ ID NO: 1. The genome may have an open reading frame 1 (ORF1) nucleotide sequence consisting of $51^{st}$ to $992^{nd}$ nucleotides of the nucleotide sequence of SEQ ID NO: 1 and an ORF2 nucleotide sequence complementary to $1037^{th}$ to $1735^{th}$ nucleotides of the nucleotide sequence of SEQ ID NO: 1. The ORF1 nucleotide sequence is a positive strand and may encode a replicase protein. The ORF2 nucleotide sequence is a negative strand and may encode a capsid protein. The ORF1 protein and ORF2 protein may respectively have amino acid sequences of SEQ ID NOS: 2 and 3.

PCV2 GC0504 viral particles may be easily obtained since PCV2 GC0504 has high proliferative activity in host cells. Thus, PCV2 GC0504 may be efficiently used to prepare attenuated circovirus vaccines. For example, PCV2 GC0504 may be prepared by cultivating PK—15 cells in 10% fetal bovine serum (FBS) supplemented a Dulbecco's modified Eagle medium (DMEM) (900 ml DMEM, 100 ml of fetal bovine serum (FBS), 100 $\mu$**g**/ml of streptomycin, 200 IU/ml of penicillin, and 100 $\mu$**g**/ml of kanamycin) to form a single layer, removing the medium therefrom, isolating the PK-15 cells therefrom by treatment with trypsin, adding $10^{4.6}$ $TCID_{50}$/0.1ml of PCV2 to a PK-15 cell suspension for inoculation, static-cultivating the cells at 37**°C** for 3 to 4 days, completely destroying the cells by freeze-thawing the cells three times, and centrifuging the cells at 2,500 rpm for 15 minutes to obtain a supernatant. In the supernatant, an average content of PCV2 GC0504 is equal to or greater than $10^{5.6}$ $TCID_{50}$/ml, for example, in the range of $10^{5.6}$ to $10^{6.0}$ $TCID_{50}$/ml. Thus, it can be seen that PCV2 GC0504 has high proliferative activity. The high proliferative activity of PCV2 GC0504 may also be obtained in the absence of proliferation enhancer, such as glucosamine.

PCV2 GC0505 may include a genome having a nucleotide sequence of SEQ ID NO: 4. The genome may have an ORF1 nucleotide sequence consisting of $51^{st}$ to $992^{nd}$ nucleotides of the nucleotide sequence of SEQ ID NO: 4 and an ORF2 nucleotide sequence complementary to $1036^{th}$ to $1734^{th}$ nucleotides of the nucleotide sequence of SEQ ID NO: 4. The ORF1 nucleotide sequence is a positive strand and may encode a replicase protein. The ORF2 nucleotide sequence is a negative strand and may encode a capsid protein. The ORF1 protein and ORF2 protein may respectively have amino acid sequences of SEQ ID NOS: 5 and 6.

PCV2 GC0505 viral particles may be easily obtained since PCV2 GC0505 has high proliferative activity in host cells. Thus, PCV2 GC0505 may be efficiently used to prepare attenuated circovirus vaccines. For example, PCV2 GC0505 may be prepared by cultivating PK-15 cells in 10% fetal bovine serum (FBS) supplemented-Dulbecco's modified Eagle medium (DMEM) (900 ml of DMEM, 100 ml of 10% fetal bovine serum (FBS), 100 $\mu$**g**/ml of streptomycin, 200 IU/ml of penicillin, and 100 $\mu$**g**/ml of kanamycin) to form a single layer, removing the medium therefrom, isolating the PK-15 cells therefrom by treatment with trypsin, adding $10^{4.6}$ $TCID_{50}$/0.1ml of PCV2 to a cell suspension for inoculation, static-cultivating the cells at 37°**C** for 3 to 4 days, completely destroying the cells by freeze-thawing the cells three times, and centrifuging the cells at 2,500 rpm for 15 minutes to obtain a supernatant. In the supernatant, an average content of PCV2 GC0505 is equal to or greater than $10^{5.6}$ $TCID_{50}$/ml, for example, in the range of $10^{5.6}$ to $10^{6.0}$ $TCID_{50}$/ml. Thus, it can be seen that PCV2 GC0505 has high proliferative activity. The high proliferative activity of PCV2 GC0505 may be obtained in the absence of proliferation enhancer, such as glucosamine.

At least one circovirus selected from the group consisting of PCV2 GC0504 and PCV2 GC0505 may be prepared from their genomic sequences by using any known method in the art. The circovirus may be produced by inserting their genomic sequence into a host cell or host animal of PCV2 and isolating infectious PCV2 from the host cell or host animal.

The host cell may be a PK-15 cell. The host animal may be a pig. For example, the circovirus may be produced by obtaining genomic DNA of PCV2 GC0504 or PCV2 GC0505 having a nucleotide sequence of SEQ ID NO: 1 or 2, introducing the DNA into a recombinant vector, inserting the recombinant vector into a host cell or host animal of PCV2, and isolating infectious PCV2 from the host cell or host animal. For example, the genomic DNA of PCV2 GC0504 or PCV2 GC0505 having a nucleotide sequence of SEQ ID NO: 1 or 2 may be obtained by DNA synthesis. The whole genomic DNA or fragments of the genomic DNA may be synthesized. When fragments of the genomic DNA are synthesized, the fragments are ligated to prepare the genomic DNA. The ligation may be performed by enzymes such as a DNA ligation enzyme and a DNA kinase. The genomic DNA of PCV2 GC0504 or PCV2 GC0505 with a length of about 1.8 kb may be easily synthesized by those of ordinary skill in the art. The synthesized genomic DNA or a vector including the genomic DNA may be inserted into the host cell or the host animal. The virus may be produced using methods disclosed in U.S. Patent Application Publication No. 2004/0253270A1 (e.g., Paragraph Nos. 0084 to 0086, Examples 2 to 5, and FIG. 1) and disclosed by Fenaux, M, et. al., (J. Vir. Jan. 2002, p.541-551), the disclosures of which are incorporated herein in their entireties by reference. If these methods are applied to the production of PCV2 GC0504 or PCV2 GC0505, the whole genome of PCV2 GC0504 or PCV2 GC0505 may be amplified by PCR using the synthesized genomic DNA as a template and a pair of forward and reverse primers having a unique restriction enzyme site, e.g., SacII site. In order to obtain DNA clone including a tandem dimer of PCV2 genome, the PCR products are ligated to a vector, for example, TAge plasmid vector (Clontech, Palo Alto, Clif) to transform E. Coli DH5a competent cell. The vector is isolated from the transformed cell to identify the existence of the whole genome and cut using the restriction enzyme, e.g., SacII, and the cut PCV2 genomic DNA is ligated using a DNA ligase to prepare a tandem dimer. Then, the tandem dimer is cloned in a pBluescript SK(+) vector (pSK) (Stratagene Inc., La Jolla, Calif.) to prepare a recombinant plasmid including a tandem dimer of PCV2 genome (PCV2 molecular DNA clone). Then, the PCV2 molecular DNA clone may be in vitro transfected into PK-15 cells. The transfected PK-15 cells are cultivated and destroyed to isolate the virus to obtain the PCV2. The PCV2 may be inoculated, for example, intranasally inoculated, into a host animal, for example, a pig, in vivo and isolated from the host animal.

The attenuated circoviruses PCV2 GC0504 and PCV2 GC0505 may not infect the host cell and do not cause pathological lesions to have immunogenicity. Alternatively, PCV2 GC0504 and PCV2 GC0505 may infect the host cell but do not cause pathological lesions to have immunogenicity. The pathological lesions may be any symptom occurring in cells, tissues, and individuals caused by the host infection by circovirus known in the art. For example, the pathological lesions may include at least one selected from the group consisting of congenital tremor, postweaning multisystemic wasting syndrome (PMWS), wasting, dyspnea, tachypnea, jaundice, diarrhea, cough, central nervous system instability, weight loss, acute vasculitis (Langohr, 2010, Vet pathol. vol 47, p140-147), breeding disease (miscarriage, stillbirth, fetal mummification, etc., Hansen, 2010, Vet Microbiol., Epub ahead of print), and porcine dermatitis nepropathy syndrome (PDNS).

The attenuation may be conducted using any method known in the art. For example, the attenuation may be conducted by repeated passages of the circoviruses in cells such as PK-15 cells or by the activation of circovirus-associated endonuclease. In addition, the attenuation may be conducted by chemical methods, for examples, by exposing the virus to formaldehyde (formalin), paraformaldehyde, β-propiolactone, ethyleneimine, or derivatives thereof or by UV-irradiation. For example, at least one virus selected from the group consisting of PCV2 GC0504 and PCV2 GC0505 is subjected to reaction with formaldehyde to be attenuated. In the reaction, the amount of formaldehyde may be in the range of 0.2 to 0.5 wt% based on the total weight of the reaction solution. The reaction may be conducted at room temperature while stirring at 100 rpm for 10 to 24 hours.

The composition may be used to treat or prevent pathological lesions caused by PCV2 infection in animals. For example, the pathological lesions may include at least one selected from the group consisting of congenital tremor, postweaning multisystemic wasting syndrome (PMWS), wasting, dyspnea, tachypnea, jaundice, diarrhea, cough, central nervous system instability, weight loss, and porcine dermatitis nepropathy syndrome (PDNS).

The amount of PCV2 GC0504 and PCV2 GC0505 contained in the composition may vary according to the type of animal, severity of disease, age of the animal, and formulation of the composition. For example, the amount of PCV2 GC0504 and PCV2 GC0505 may be equal to or greater than $10^{4.5}$ $TCID_{50}$, for example, in the range of $10^{4.5}$ $TCID_{50}$ to $10^{6.5}$ $TCID_{50}$ at a single dose.

In the composition, the circovirus to be prevented or treated infections may be selected from the group consisting of PCV2a and PCV2b. For example, the circovirus may include PCV2a and PCV2b. PCV2 is classified into two genotypes, PCV2a and PCV2b, but the PCV2a and PCV2b are not classified into substrains. Thus, the composition including the attenuated PCV2 GC0504 and PCV2 GC0505 according to an embodiment of the present invention may defend viruses belonging to PCV2a and PCV2b, i.e., all PCV2.

The composition may further include a veterinarily acceptable carrier. The carrier may be any carrier commonly used in the art to maintain immunogenicity of the composition. For example, the carrier may include at least one selected from the group consisting of a diluent, an excipient, an adjuvant, and a freeze-drying stabilizer. The diluent may include at least one selected from the group consisting of water such as sterilized water and a buffer solution. The adjuvant may be any adjuvant commonly used in the art to improve immunogenicity of PCV2 GC0504 and PCV2 GC0505. For example, the adjuvant may include at least one selected from the group consisting of aluminium hydroxide, saponin, polyphosphazene, and montanide gel™. The freeze-drying stabilizer may be carbohydrates such as sorbitol, mannitol, starch, sucrose, dextran, or glucose, proteins such as albumin or casein, derivatives of these compounds, or buffers such as alkali metal phosphates. The amount of the carrier may vary according to the type of the carrier as well known by those of ordinary skill in the art. The composition may include 5 to 15% of the carrier based on the total weight of the composition.

According to the current embodiment, the composition may include $10^{4.5}$ $TCID_{50}$ or greater, e.g., $10^{4.5}$ $TCID_{50}$ to $10^{7.5}$ $TCID_{50}$, $10^{4.5}$ $TCID_{50}$ to $10^{6.5}$ $TCID_{50}$, or $10^{6.0}$ $TCID_{50}$ to $10^{7.0}$ $TCID_{50}$, of PCV2 GC0504 and PCV2 GC0505, respectively per 1 unit dosage of the composition, and 5% to 10% of montanide gel™ based on the total weight of the composition. The montanide gel™ (SEPPIC, Paris, France) consists of a synthetic polymer and a hydrophilic gel.

The composition may be an immunogenic composition in any formulation known in the art. The composition may be an oral or parenteral formulation. The formulation of the composition may be a freeze-dried formulation, granule, powder, liquid, tablet, capsule, dried syrup, or injection. The freeze-dried formulation may be dissolved in a sterilized liquid carrier, e.g., sterilized water, before use.

The composition may be administered using any method commonly used in the art to administer an immunogenic composition. For example, the composition may be administered into individuals via a route such as an intravenous, intramuscular, oral, transdermal, mucosal, intranasal, intratracheal, or subcutaneous route. The administration may be a systematic administration or a topical administration.

In the composition, the animal may be selected from the group consisting of pig, mouse, and guinea pig.

One or more embodiments of the present invention provide a method of producing an attenuated circovirus, the method including reacting at least one virus selected from the group consisting of PCV2 GC0504 and PCV2 GC0505 with formalin to obtain attenuated circovirus.

The reaction may be conducted by adding the virus into a reaction solution including formalin and maintaining the reaction solution. The amount of formalin may be in the range of 0.2 to 0.5 wt% based on the total weight of the reaction solution. The reaction may be conducted at room temperature. The reaction may be conducted for 10 to 24 hours. The reaction may be conducted while stirring at 100 rpm.

One or more embodiments of the present invention provide a method of treating or preventing circovirus infection in animals, the method including administering the composition having an effective amount into the animals.

The composition may be administered using any method commonly used in the art to administer an immunogenic composition. The composition may be orally or parenterally administered. For example, the composition may be administered into individuals via a route such as an intravenous, intramuscular, oral, transdermal, mucosal, intranasal, intratracheal, rectal, or subcutaneous route. The administration may be a systematic administration or a topical administration. The administration may be conducted by injection or infusion.

The composition may be administered once or more, e.g., twice or more. The composition may be administered twice. For example, the composition may be administered once and administered again after two weeks from the first administration. The animal may be selected from the group consisting of pig, mouse, and guinea pig.

The "effective amount" refers to an amount effective for preventing or treating PCV2 infection in animals. The treatment of prevention includes removing and reducing lesions caused by PCV2 infection in addition to preventing PCV2 infection in animals. The effective amount may vary according to the type of animal, severity of disease, age of the animal, and formulation of the composition. For example, the effective amount of PCV2 GC0504 and PCV2 GC0505 may include $10^{4.5}$ TCIDso or greater, for example, $10^{4.5}$ $TCID_{50}$ to $10^{7.5}$ $TCID_{50}$, $10^{4.5}$ $TCID_{50}$ to $10^{6.5}$ $TCID_{50}$, or $10^{6.0}$ $TCID_{50}$ to $10^{7.0}$ $TCID_{50}$, of PCV2 GC0504 and PCV2 GC0505, respectively at a single dose.

The composition is defined above. The animal may be any animal in which pathological lesions are caused by PCV2 infection. For example, the animal may be selected from the group consisting of pig, mouse, and guinea pig.

One or more embodiments of the present invention provide circovirus PCV2 GC0504 including a genome having a nucleotide sequence of SEQ ID NO: 1.

The genome may have an ORF1 nucleotide sequence consisting of 51st to 992nd nucleotides of the nucleotide sequence of SEQ ID NO: 1 and an ORF2 nucleotide sequence complementary to 1037th to 1735th nucleotides of the nucleotide sequence of SEQ ID NO: 1. The ORF1 nucleotide sequence is a positive strand and may encode a replicase protein. The ORF2 nucleotide sequence is a negative strand and may encode a capsid protein. The ORF1 protein and ORF2 protein may respectively have amino acid sequences of SEQ ID NOS: 2 and 3.

PCV2 GC0504 may include any viral particles expressed by the genomic sequence of SEQ ID NO: 1. The circovirus PCV2 GC0504 may include at least one amino acid sequence selected from the group consisting of ORF1 and ORF2 having amino acid sequences of SEQ ID NOS: 2 and 3. The circovirus PCV2 GC0504 is a PCV2-2a virus.

Due to high proliferative activity in a host cell, PCV2 GC0504 may be efficiently used to prepare attenuated circovirus vaccine. For example, PCV2 GC0504 may be prepared by cultivating PK-15 cells in 10% fetal bovine serum (FBS) supplemented-Dulbecco's modified Eagle medium (DMEM) (900 ml of DMEM,100 ml of 10% fetal bovine serum (FBS), 100 μg/ml of streptomycin, 200 IU/ml of penicillin, and 100 μg/ml of kanamycin) to form a single layer, removing the medium therefrom, isolating the PK-15 cells therefrom by treatment with trypsin, adding $10^{4.6}$ $TCID_{50}$/0.1ml of PCV2 to a cell suspension for inoculation, static-cultivating the cells at 37 for 3 to 4 days, completely destroying the cells by freeze-thawing three times, and centrifuging the cells at 2,500 rpm for 15 minutes to obtain a supernatant. In the supernatant, an average amount of PCV2 GC0504 is equal to or greater than $10^{5.6}$ $TCID_{50}$/ml, for example, in the range of $10^{5.6}$ to $10^{6.0}$ $TCID_{50}$/ml. Thus, it can be seen that PCV2 GC0504 has high proliferative activity. PCV2 GC0504 is a virus belonging to PCV2-2a.

The attenuated circovirus PCV2 GC0504 may have immunogenicity against various species of PCV2a strains.

One or more embodiments of the present invention provide circovirus PCV2 GC0505 including a genome having a nucleotide sequence of SEQ ID NO: 2.

PCV2 GC0505 may include a genome having a nucleotide sequence of SEQ ID NO: 4. The genome may have an ORF1 nucleotide sequence consisting of $51^{st}$ to $992^{nd}$ nucleotides of the nucleotide sequence of SEQ ID NO: 4 and an ORF2 nucleotide sequence complementary to $1036^{th}$ to $1734^{th}$ nucleotides of the nucleotide sequence of SEQ ID NO: 4. The ORF1 nucleotide sequence is a positive strand and may encode a replicase protein. The ORF2 nucleotide sequence is a negative strand and may encode a capsid protein. The ORF1 protein and ORF2 protein may respectively have amino acid sequences of SEQ ID NOS: 5 and 6. PCV2 GC0505 may include any viral particles expressed by the genomic sequence of SEQ ID NO: 4. PCV2 GC0505 may include at least one amino acid sequence selected from the group consisting of ORF1 and ORF2 having amino acid sequences of SEQ ID NOS: 5 and 6. PCV2 GC0505 is a virus belonging to PCV2-2b.

Due to high proliferative activity in a host cell, PCV2 GC0505 may be efficiently used to prepare attenuated circovirus vaccine. For example, PCV2 GC0505 may be prepared by cultivating PK-15 cells in 10% fetal bovine serum (FBS) supplemented- Dulbecco's modified Eagle medium (DMEM) (900 ml of DMEM, 100 ml of 8% fetal bovine serum (FBS), 100 $\mu$**g**/ml of streptomycin, 200 IU/ml of penicillin, and 100 $\mu$**g**/ml of kanamycin) to form a single layer, removing the medium therefrom, isolating the PK-15 cells therefrom by treatment with trypsin, adding $10^{4.6}$ $TCID_{50}$/0.1ml of PCV2 to a cell suspension for inoculation, static-cultivating the cells at 37 for 3 to 4 days, completely destroying by freeze-thawing three times, and centrifuging the cells at 2,500 rpm for 15 minutes to obtain a supernatant. In the supernatant, an average amount of PCV2 GC0505 is equal to or greater than $10^{5.6}$ $TCID_{50}$/ml, for example, in the range of $10^{5.6}$ to $10^{6.0}$ $TCID_{50}$/ml. Thus, it can be seen that PCV2 GC0505 has high proliferative activity.

The attenuated circovirus PCV2 GC0505 may have immunogenicity against various species of PCV2b strains.

Each of PCV2 GC0504 and PCV2 GC0505 may be replicated in vitro or in vivo in a porcine cell line. The cell line may be an immortalized cell line. The cell line may be selected from the group consisting of a porcine kidney epithelial cell lines PK-15 and SK, monomyeloid cell line 3D4/31, and testicular cell line ST.

The nucleotide sequences described herein are attached hereto as a sequence list, the disclosure of which is incorporated herein in its entirety by reference.

The present disclosure will be described in further detail with reference to the following examples. These examples are for illustrative purposes only and are not intended to limit the scope of the invention.

Example 1

### 1. Isolation of Virus and Identification of Proliferative Activity

PCV was isolated from specimen of pigs requested between 2005 to 2007 using PCR and indirect immunofluorescence assay (IFA). PK-15 cells were infected with the PCV isolate and cultivated to measure the number of proliferated viral particles, and then a PCV isolate having the highest proliferative activity was selected.

First, PK-15 cells were cultivated in 10% fetal bovine serum (FBS) supplemented-Dulbecco's modified Eagle medium (DMEM) (900 ml of DMEM, 100 ml of 10% fetal bovine serum (FBS), 100 $\mu$**g**/ml of streptomycin, 200 IU/ml of penicillin, and 100 $\mu$**g**/ml of kanamycin) at 37 to form a single layer. Then, the medium was removed from the PK-15 cells having the single layer, and the PK-15 cells were isolated therefrom by treatment with trypsin. $10^{4.5}$ $TCID_{50}$/0.1ml of PCV2 was added to a virus-containing cell suspension for inoculation, and the cells were static-cultivated at 37 for 3 to 4 days. The cultivated cells were freeze-thawed three times to completely destroy the cells and centrifuged the cells at 2,500 rpm for 15 minutes. The amount of PCV2 contained in a supernatant obtained by the centrifugation was measured using PCR and IFA. As a result, it was identified that two PCV2 isolates had high virus contents. Then, the two isolates were referred to as PCV2 GC0504 and PCV2 GC0505. PCV2 GC0504 is a PCV2-2a virus and has a genomic sequence of SEQ ID NO: 1. PCV2 GC0505 is a PCV2-2b virus and has a genomic sequence of SEQ ID NO: 2. PCV2 GC0504 and PCV2 GC0505 are replicated such that an average amount thereof is in the range of $10^{5.6}$ to $10^{7.0}$ $TCID_{50}$/ml in a culture medium. It was identified that PCV2 GC0504 and PCV2 GC0505 have more than10 times higher proliferative activity than conventional PCV2. Thus, at least one virus selected from the group consisting of PCV2 GC0504 and PCV2 GC0505 may efficiently provide viruses required for preparing attenuated or inactivated vaccine. Thus, the obtained PCV2 (bulk virus) was used to attenuate the following viruses.

In this regard, the PCR was real time-PCR. In the real time-PCR, a SYBR green manufactured by Takara was used as a reagent. A sample obtained by diluting $10^{6.0}$ $TCID_{50}$/ml of PCV2 GC0505 by ten times was used as a template and primers specific to ORF2 of PCV2 (5'-TAG GTT AGG GCT GTG GCC TT-3' forward primer, SEQ ID NO: 7 and 5'-CCG CAC CTT CGG ATA TAC TG-3' reverse primer, SEQ ID NO: 8) were used. The sample was maintained at 94 for 10 seconds, at 94 for 5 seconds, and at 60 for 40 seconds, and this process was repeated 40 times. The amplified product was anticipated to be 263 bp. As a result, the virus was successfully detected at a concentration of 10 $TCID_{50}$/ml (FIG. 1). FIG. 1 is a graph illustrating results of real time-polymerase chain reaction (PCR) of PCV2 having various concentrations.

The relationship between Ct value measured by the real time-PCR and virus titer measured by IFA is defined as in Formula 1 below.

Formula 1

$$y=4.1291x+4.458$$

In Formula 1, y is Ct value, x is IFA titer ($log_{10}$), and the unit is $TCID_{50}$/0.1 ml. According to Formula 1, the correlation coefficient ($R^2$) is 0.9988, indicating about 99.9% reliability. Since the real time-PCR showed high sensitivity capable of detecting the virus concentration of 10 $TCID_{50}$/1 ml and was quickly performed, it was efficiently used to measure the virus.

PCV2-specific antibody was measured using sandwich indirect enzyme-linked immunosorbent assay (ELISA).

First, the PCV2-specific antibody was diluted in a coating buffer including 2.93 g of $NaHCO_3$, 1.59 g of $Na_2CO_3$, 1000 ml of water, and 0.5 M carbonate buffer (pH 9.6) to a concentration of 5 to 10 μg/well, and 100 μℓ of the diluted solution was added to each well of an ELISA plate and adsorbed thereto at 37 for 2 hours. The ELISA plate to which the antibody was adsorbed was washed three times with PBS-T. 200 μℓ of a blocking buffer (BSA 1-2 g, PBS 100 ml: 1-2% BSA) was added to each well and the plate was maintained at 37 for 2 hours. The plate was washed three times. Then, the PCV2 antigen was diluted 10 times to 20 times with PBS, and the diluted solution was added to each well and the plate was maintained at 37 for 1 hour. The plate was washed three times. Then a diluted sample (serum) obtained by diluting the sample (serum) with a dilution buffer including 8.0 g of NaCl, 0.2 g of anhydrous $KH_2PO_4$, 1.19 g of anhydrous $Na_2HPO_4$, 0.2 g of KCl, and 1000 ml of water (pH 7.2-7.4) to a ratio of 1:50 was added to each well of the plate in which the antigen was sensitized, and the plate was maintained at 37 for 1 hour. The plate was washed five times with a washing buffer (Tween 20 0.5 ml, PBS 1,000 ml: 0.05% PBST), and 100 μℓ of anti-GP IgG HRP conjugate diluted with the dilution buffer was added thereto. The mixture was maintained at 37 for 1 hour. The plate was washed five times, and 100 μℓ of OPD substrate was added to each well of the plate. The plate was maintained at room temperature for 10 minutes, and 50 μℓ of a stop solution (2.5M $H_2SO_4$) was added thereto, and then absorbance was measured at 492 nm.

The isolated PCV2 GC0504 and PCV2 GC0505 were inoculated into PK-15 cells having a single layer, and the cells were cultivated at 37 for 72 hours to harvest a virus-infected solution. The solution was centrifuged at 2,500 rpm for 15 minutes, and the supernatant or freeze-dried supernatant was preserved at a temperature equal to or less than -80.

**2. Attenuation of Virus and Preparation of Immunogenic Composition Including Attenuated Virus**

**(2.1) Attenuation of Virus**

Among the bulk virus harvested Operation 1 above, bulk virus having a concentration of $10^{6.0}$ $TCID_{50}$/ml or greater was used for the attenuation. Formalin was added in a concentration of 0.3% to the bulk virus in 10% FBS supplemented- DMEM (900 ml of a DMEM, 100 ml of 10% FBS, 100 μ**g**/ml of streptomycin, 200 IU/ml of penicillin, and 100 μ**g**/ml of kanamycin), and the medium was maintained at room temperature for 24 hours to attenuate PCV2 GC0504 and PCV2 GC0505. The attenuated PCV2 GC0504 and PCV2 GC0505 were used as a bulk material to prepare an immunogenic composition.

**(2.2) Preparation of immunogenic composition**

The concentration of each of PCV2 GC0504 and PCV2 GC0505 prepared in Operation (2.1) was respectively set to $10^{6.0}$ $TCID_{50}$/ml, and PCV2 GC0504 and PCV2 GC0505 are mixed at a ratio of 45: 45. Motanide gel™ was added thereto to a concentration of 10% (w/w) and the mixture was stirred at room temperature at 100 rpm for more than 1 hour. The pH of the prepared immunogenic composition was maintained in the range of 6.0-8.0, and the concentration of formalin was maintained to be equal to or less than 0.3 wt%. The immunogenic composition prepared herein will be used in the following processes unless otherwise stated. Hereinafter, the immunogenic composition is also referred to as a "vaccine".

**(3) Identification of Attenuation and Stability**

**(3.1) Identification of Attenuation**

The attenuated sample was centrifuged and an appropriate amount of the sample was added to a dialysis membrane. The sample was dialyzed overnight in a phosphate buffer solution (PBS) in an amount 100 times or greater than the sample at 4 to remove the attenuating agent. The resultant was used for experiments.

Primary porcine kidney cells or PK-15 cells were proliferated in a flask (25 **$cm^2$**) for tissue cultivation. The primary cells were cells cultured for 4 to 7 days, and it was identified that the PK-15 cells were not infected with PCV1 before experiments. The medium was removed from the proliferated cells, and 1 **Mℓ** of the sample was inoculated into the tissue-cultivated cells and sensitized at 37 for 1 hour. Then, a new culture medium for maintaining the cells was added thereto, and the cells were further cultivated for 7 days to identify cytopathic effect (CPE). When the supernatant was removed, the proliferation of the porcine circovirus was identified using IFA.

As a result, CPE caused by the porcine circovirus or any other virus was not observed in the primary porcine kidney cells or PK-15 cells. In addition, in the supernatant, hemagglutination (HA) against chickens and pigs blood cells was not observed, and no virus was detected by IFA.

(3.2) Identification of Stability

8 mice having weights in the range of 15 to 20 g and 4 guinea pigs having weights in the range of 300 to 400 g were used. In addition, 2 healthy young pigs between 3 to 5 weeks of age which were not inoculated with PCV2-associated vaccine and not exposed to PCV2 were used.

0.5 ml ($10^{6.0}$ $TCID_{50}$/ml) of the composition was intraperitoneally inoculated into the 8 mice, 2 **Mℓ** ($10^{6.0}$ $TCID_{50}$/ml) of the composition was intramuscularly or subcutaneously inoculated into the 4 guinea pigs, and 2 **Mℓ** of the composition was intraperitoneally inoculated into the two young pigs, and the animals were observed for 7 days whether they were alive. After a single dose of $10^{6.0}$ $TCID_{50}$/ml was inoculated into muscle on the right of the neck of the 2 young pigs, the 2 young pigs were observed whether the pigs had hypersensitive reactions or fever within 1 to 2 hours and whether the pigs survived without side effects such as purulence or necrosis for 21 days. As a result of inoculating the composition with a varying lots into the mice, guinea pigs, and young pigs, they were alive without any side effect during the experiments.

**(3.3) Antibody Titer**

The immunogenic composition was administered into animals, and antibody titer of serums were measured. The antibody titer was measured using sandwich indirect ELISA or IFA.

10 guinea pigs having weights in the range of 300 to 350 g were prepared. 8 of them were used as an inoculation group, and 2 of them were used as a control group. 0.5 ml ($10^{6.0}$ $TCID_{50}$/ml) of the composition was subcutaneously inoculated into the inoculation group. After 3 weeks, blood samples of the 10 guinea pigs were taken, and antibody titers thereof were measured using IFA or sandwich indirect ELISA.

The antibody titer measured using sandwich indirect ELISA was calculated by dividing an average absorbance (O.D.) of the inoculation group T by an average absorbance (O.D.) of the control group C. When the T/C was 2.0 or higher, the T/C for an average antibody titer of the inoculation group obtained by IFA was 128 times or greater, and the control group was negative, it was determined that the immunogenic composition was appropriate. The antibody titers measured using ELISA are shown in Table 1 below.

Table 1

| No. | Group | Absorbance | Absorbance (T/C) |
|---|---|---|---|
| 1 | Inoculation | 0.88 | 2.9 |
| | Control | 0.30 | |
| 2 | Inoculation | 0.82 | 3.4 |
| | Control | 0.24 | |

(continued)

| No. | Group | Absorbance | Absorbance (T/C) |
|---|---|---|---|
| 3 | Inoculation | 0.87 | 3.0 |
| | Control | 0.29 | |
| 4 | Inoculation | 0.88 | 2.9 |
| | Control | 0.30 | |

As shown in Table 1, the immunogenic composition has immunogenicity suitable for the inoculation group (experimental group) compared to the control group.

**3. Identification of Effectiveness of Immunogenic Composition**

Here, a minimum amount of the immunogenic composition required for inducing immunity in animals (amount of antigen required for minimum immunity) is determined, and effectiveness such as immunity-inducing ability and weight gain rate when the immunogenic composition is inoculated into animals is identified.

The same immunogenic composition was used except that PCV2 titers of $10^{3.5}$, $10^{4.5}$, $10^{5.5}$, and $10^{6.5}$ $TCID_{50}$/ml were used to measure the amount of antigen required for minimum immunity (4 times repeated). The immunogenic composition was used to measure the effectiveness.

Experiments to determine the amount of antigen required for minimum immunity were performed using young pigs. PCV2 vaccines having antigen contents of $10^{3.5}$, $10^{4.5}$, $10^{5.5}$, and $10^{6.5}$ $TCID_{50}$/ml were inoculated twice into fifteen 3-week old young pigs which are negative against porcine circoviruses at 2-week intervals, and three young pigs were used as a control group. Blood samples of the young pigs were taken before the inoculation and after two weeks from the second inoculation to measure antibody titers of PCV2.

The effectiveness of the immunogenic composition was tested using young pigs. The immunogenic composition was inoculated twice into four 3-week old young pigs which were negative against porcine circovirus at 2-week intervals, and two young pigs were used as a control group. Blood samples of the young pigs were taken after 2 weeks, 4 weeks, 2 months, 3 months, and 4 months after the second inoculation, and PCV antibody titer was measured using ELISA and serum neutralizing (SN) antibody titer using IFA were measured as described above (Fort, et al., 2007:Vet Microbiol 125, p244-255). The antibody titer obtained using ELISA indicates all antibodies induced by the antigen, and the SN antibody titer indicates a serum neutralizing antibody titer that is an antibody level capable of neutralizing pathogens such as virus among the antibodies existing in the serum.

In addition, the effect of the immunogenic composition to a weight gain rate of the young pigs was measured. Weights of the young pigs were measured before the first inoculation, before the second inoculation, after two weeks from the second inoculation, and after six weeks from the second inoculation to identify the weight gain rate.

As results of measuring the amount of the antigen required for the minimum immunity and the antibody titer after the second inoculation using ELISA, the change of the antibody titer was identified at $10^{3.5}$ $TCID_{50}$/ml. However, when the antigen content was greater than $10^{4.5}$ $TCID_{50}$/ml, the antibody titer was significantly increased ($p<0.05$) when compared to the control group (Table 2).

Table 2 Immunogenicity according to PCV2 content (ELISA)

| Time | Antibody titer & significance | PCV2 content ($TCID_{50}$/ml) | | | | Control |
|---|---|---|---|---|---|---|
| | | $10^{3.5}$ | $10^{4.5}$ | $10^{5.5}$ | $10^{6.5}$ | - |
| Before | M* | 0.59 | 0.65 | 0.45 | 0.57 | 0.47 |
| inoculation | Significance | 0.24 | 0.46 | 0.34 | 0.39 | |
| After 2 weeks from 2nd inoculation | M* | 0.49 | 0.69 | 0.73 | 0.71 | 0.37 |
| | Significance | 0.18 | <0.05 | <0.05 | <0.05 | |
| M* is a geometric mean antibody titer. | | | | | | |

Table 3 shows antibody titers of young pigs into which the immunogenic composition was inoculated.

Table 3 Antibody titers of young pigs against PCV2 after the inoculation of the immunogenic composition.

| Time | Antibody titer & significance | PCV2 | | | |
|---|---|---|---|---|---|
| | | v[a] | | NV[b] | |
| | | ELISA Value | SN titer | ELISA Value | SN titer |
| Before 1st inoculation | M[c] | 0.31 | <2 | 0.35 | <2 |
| | Significance | 0.34 | | | |
| Before 2nd inoculation | M[c] | 0.47 | <2 | 0.36 | <2 |
| | Significance | 0.19 | | | |
| After 2 weeks from2nd inoculation | M[c] | 0.77 | 48 | 0.32 | <2 |
| | Significance | <0.05 | <0.05 | | |
| After 4 weeks from 2nd inoculation | M[c] | 0.84 | 68 | 0.37 | <2 |
| | Significance | <0.05 | <0.05 | | |
| After 2 months from 2nd inoculation | M[c] | 0.81 | 64 | 0.35 | <2 |
| | Significance | <0.05 | <0.05 | | |
| After 3 months from 2nd inoculation | M[c] | 0.78 | 62 | 0.37 | <2 |
| | Significance | <0.05 | <0.05 | | |
| After 4 months from 2nd inoculation | M[c] | 0.80 | 62 | 0.35 | <2 |
| | Significance | <0.05 | <0.05 | | |

M* is a mean value.
a: V (vaccinated group) (n=4)
b: NV (non-vaccinated group) (n=2)

Tables 4 and 5 show weights of young pigs inoculated with the immunogenic composition. Tables 4 and 5 show weights of the inoculation group and control group before the first inoculation, before the second inoculation, after two weeks from the second inoculation, and after 6 weeks from the second inoculation. An average weight of the inoculation group after two weeks from the second inoculation, i.e., seven-week old, was greater than that of the control group by about 1 kg, and an average weight of the inoculation group at the age of 13 weeks was greater than that of the control group by about 4 kg.

Table 4

| Individual No. | Before inoculation, 3-week old | Before 2nd inoculation, 5-week old | After 2 weeks from 2nd inoculation, 7-week old | After 6 weeks from 2nd inoculation, 13-week old |
|---|---|---|---|---|
| 1 | 5.5 | 8.3 | 15.6 | 41.3 |
| 2 | 4.6 | 7.9 | 15.7 | 38.2 |
| 3 | 4.8 | 8.1 | 14.9 | 39.5 |
| 4 | 6.1 | 9.2 | 16.2 | 45.2 |
| 5 | 4.9 | 8.5 | 15.6 | 47.9 |
| 6 | 5.3 | 8.8 | 15.3 | 38.3 |
| 7 | 4.5 | 7.6 | 16.1 | 35.2 |
| 8 | 4.8 | 8.3 | 15.9 | 40.6 |
| 9 | 5.5 | 8.7 | 14.5 | 41.2 |
| 10 | 5.3 | 7.1 | 16.9 | 43.5 |
| Average | 5.13 | 8.25 | 15.67 | 41.09 |

Table 4 shows weights of the inoculation group (kg).

Table 5

| Individual No. | 3-week old | 5-week old | 7-week old | 13-week old |
|---|---|---|---|---|
| 1 | 5.5 | 8.5 | 14.5 | 37.5 |
| 2 | 4.8 | 8.8 | 14.3 | 37.3 |
| 3 | 5.1 | 8.2 | 14.7 | 38.2 |
| 4 | 4.7 | 7.9 | 14.8 | 39.1 |
| 5 | 5.2 | 8.3 | 15.2 | 34.2 |
| 6 | 5.1 | 8.9 | 14.1 | 38.1 |
| 7 | 4.3 | 7.6 | 14.6 | 37.4 |
| 8 | 5.3 | 7.3 | 14.8 | 38.2 |
| 9 | 5.1 | 7.8 | 14.6 | 39.1 |
| 10 | 4.9 | 7.4 | 15.2 | 35.9 |
| Average | 5.00 | 8.07 | 14.68 | 37.50 |

Table 5 shows weights of the control group (kg).

In order to identify the effectiveness of the immunogenic composition including attenuated porcine circovirus, antibody titers were measured after the immunogenic composition was inoculated into animals. As a result, in the inoculation group, an increase in the antibody titer was greater than that in the control group. When the immunogenic composition having various concentrations was inoculated into the young pigs, antibody titers significantly increased at a concentration equal to or greater than $10^{4.5}TCID_{50}$/ml. Thus, the concentration of the attenuated virus contained in the immunogenic composition needs to be at least $10^{4.5}TCID_{50}$/ml. According to the immunogenicity test, the inoculated young pigs showed far greater antibody titers than those of the control group. According to the weight gain rate test, the young pigs of the inoculation group showed greater weight gain rates than those the control group.

**4. Challenge-Inoculation Test of Young Pig**

The immunogenic composition was inoculated into young pigs, and PCV2 was inoculated into the pigs to identify whether defense capability of the pigs was improved by the immunogenic composition.

The immunogenic composition was prepared according to Operation (2.2) above. Fourteen 3-week old young pigs were used for the challenge-inoculation. PCV2 strain isolated from a veterinary medicine (Green Cross Corp.) was used as a virus strain for challenge-inoculation of the young pig. PCV2 was cultivated in PK-15 cells at 37 for 48 hours and centrifuged at 500 rpm for 10 minutes. The supernatant was diluted to a concentration of $10^{5.0}$ $TCID_{50}$/ml.

Among the fourteen 3-week old specific pathogen free (SPF) young pigs used to identify their defense capability, 1 ml ($10^{6.0}$ $TCID_{50}$/ml) of the immunogenic composition was intramuscularly inoculated into 8 young pigs twice at 2-week intervals (experimental group), and the other 6 young pigs were not vaccinated (control group).

After two weeks from the second inoculation, the experimental group and the control group were respectively classified into two sub-groups, and challenge-inoculation was conducted with the prepared virus strain. Then, clinical symptoms were observed for 14 days.

- First experimental group (PCV2 challenge-inoculation): In the first experimental group, 1 ml of the immunogenic composition was intramuscularly inoculated into four 3-week old young pigs twice at 2-week intervals, and PCV2 was challenge-inoculated after two weeks therefrom (at the age of 7 weeks).
- First control group (PCV2 challenge-inoculation): In the first control group, two 3-week old young pigs were raised, and PCV2 was challenge-inoculated into the pigs at the age of 7 weeks.
- Second experimental group (PCV2+PRRSV challenge-inoculation): In the second experimental group, 1 ml of the immunogenic composition was intramuscularly inoculated into four 3-week old young pigs twice at 2-week intervals, and PCV2 and PRRSV was challenge-inoculated after two weeks therefrom (at the age of 7 weeks).
- Second control group (PCV2+PRRSV challenge-inoculation): In the second control group, two 3-week old young pigs were raised, and PCV2 and PRRSV were challenge-inoculated into the pigs at the age of 7 weeks.
- No-inoculation control group: In the no-inoculation control group, two 3-week old young pigs were not vaccinated and not inoculated at seven-week old.

In addition, it was identified whether PCV2 was eluted from the challenge-inoculated young pigs. The elution of the virus was identified by swabbing nasal cavity and rectum with sterilized cotton sticks at the day of challenge-inoculation and after 3 days, 7 days, 10 days, 14 days, 17 days, 20 days, 25 days, and 31 days from the challenge-inoculation and measuring the virus content. The PCV2 content was measured using real time-PCR (SYBR Premix Ex Taq 2X, TAKARA™). DNA extracted from 100 µℓ of PBS-derived samples obtained from the nasal cavity and rectum was used as a template and real time-PCR was conducted using PCR primers described below and under the following conditions. An IFA titer was obtained using the Ct value obtained using the real time-PCR based on the relationship between the Ct value and the IFA, i.e., using Formula 1.

The real-time PCR was conducted by using primers specific to ORF2 of PCV2 (5'-TAG GTT AGG GCT GTG GCC TT-3' forward primer, SEQ ID NO: 7, and 5'-CCG CAC CTT CGG ATA TAC TG-3' reverse primer, SEQ ID NO: 8) at 95 for 10 seconds, at 95 for 5 seconds, and at 60 for 40 seconds, and this process was repeated 30 time, and then the resultant was maintained at 95 for 1 minutes, at 55 for 30 seconds, and at 95 for 30 seconds. The amplified product was anticipated to be 263 bp. The mixture for the real time-PCR had a total volume of 25 µℓ and included 9.5 µℓ of distilled water, 12.5 µℓ of SYBR Premix Ex Taq 2X, 0.5 µℓ of the forward primer (10 pmol), 0.5 µℓ of the reverse primer (10 pmol), and 2 µℓ of the template (DNA).

As a result of the challenge-inoculation, there were no particular symptoms in the first experimental group into which PCV2 was challenge-inoculated and the second experimental group into which PCV2 and PRRSV were challenge-inoculated. However, porcine circovirus associated disease (PCVAD) such as eye mucus and diarrhea were observed in the control group into which the immunogenic composition was not inoculated but the PCV2 was inoculated. In particular, typical PCV2-associated symptoms such as depression, watery diarrhea, rough hair, atrophy, and enlarged lymph nodes were observed in the first control group into which PCV2 was challenge-inoculated and in the second control group into which PCV2 and PRRSV were simultaneously challenge-inoculated after one week from the challenge-inoculation. In the second control group, one of the young pig was dead at the $11^{th}$ day from the challenge-inoculation. The experimental group into which the immunogenic composition was inoculated and the no-inoculation control group exhibited normal health conditions.

Table 6 shows clinical symptoms of the first experimental group into which PCV2 was challenge-inoculated after the challenge-inoculation.

Table 6

| | | No. of individual having clinical symptoms | | | | | | | | | | | | | |
|---|---|---|---|---|---|---|---|---|---|---|---|---|---|---|---|
| | DPI | 1 | 2 | 3 | 4 | 5 | 6 | 7 | 8 | 9 | 10 | 11 | 12 | 13 | 14 |
| Fever | Normal | 3 | 4 | 4 | 4 | 4 | 4 | 4 | 4 | 4 | 4 | 4 | 4 | 4 | 4 |
| | Depressed | 1 | 0 | 0 | 0 | 0 | 0 | 0 | 0 | 0 | 0 | 0 | 0 | 0 | 0 |
| | High fever Normal | 0 4 | 0 4 | 0 4 | 0 4 | 0 4 | 0 4 | 0 4 | 0 4 | 0 4 | 0 4 | 0 4 | 0 4 | 0 4 | 0 4 |
| Breathing | Increase | 0 | 0 | 0 | 0 | 0 | 0 | 0 | 0 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| Cough | Normal | 4 | 4 | 4 | 4 | 4 | 4 | 4 | 4 | 4 | 4 | 4 | 4 | 4 | 4 |
| | Intermittence | 0 | 0 | 0 | 0 | 0 | 0 | 0 | 0 | 0 | 0 | 0 | 0 | 0 | 0 |
| | Severe | 0 | 0 | 0 | 0 | 0 | 0 | 0 | 0 | 0 | 0 | 0 | 0 | 0 | 0 |
| Skin rashes | Normal | 4 | 4 | 4 | 4 | 4 | 4 | 4 | 4 | 4 | 4 | 4 | 4 | 4 | 4 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 4 | 4 | 4 | 4 | 4 | 4 | 4 | 4 | 4 | 4 | 4 | 4 | 4 | 4 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |

(continued)

| | | No. of individual having clinical symptoms | | | | | | | | | | | | | |
|---|---|---|---|---|---|---|---|---|---|---|---|---|---|---|---|
| | DPI | 1 | 2 | 3 | 4 | 5 | 6 | 7 | 8 | 9 | 10 | 11 | 12 | 13 | 14 |
| Diarrhea | Normal | 4 | 4 | 4 | 4 | 4 | 4 | 4 | 4 | 4 | 4 | 4 | 4 | 4 | 4 |
| | Watery diarrhea | 0 | 0 | 0 | 0 | 0 | 0 | 0 | 0 | 0 | 0 | 0 | 0 | 0 | 0 |
| | Diarrhea | 0 | 0 | 0 | 0 | 0 | 0 | 0 | 0 | 0 | 0 | 0 | 0 | 0 | 0 |

Table 7 shows clinical symptoms of the first control group into which PCV2 was challenge-inoculated (non-vaccinated group) after the challenge-inoculation.

Table 7

| | | No- of individual having clinical symptoms | | | | | | | | | | | | | |
|---|---|---|---|---|---|---|---|---|---|---|---|---|---|---|---|
| | DPI | 1 | 2 | 3 | 4 | 5 | 6 | 7 | 8 | 9 | 10 | 11 | 12 | 13 | 14 |
| Fever | Normal | 1 | 1 | 1 | 1 | 2 | 2 | 2 | 2 | 2 | 2 | 2 | 2 | 2 | 2 |
| | Depressed | 1 | 1 | 1 | 1 | 0 | 0 | 0 | 0 | 0 | 0 | 0 | 0 | 0 | 0 |
| | High fever | 0 | 0 | 0 | 0 | 0 | 0 | 0 | 0 | 0 | 0 | 0 | 0 | 0 | 0 |
| Breathing | Normal | 2 | 2 | 2 | 2 | 2 | 2 | 2 | 2 | 2 | 2 | 2 | 2 | 2 | 2 |
| | Increase | 0 | 0 | 0 | 0 | 0 | 0 | 0 | 0 | 0 | 0 | 0 | 0 | 0 | 0 |
| | Opening uth | 0 | 0 | 0 | 0 | 0 | 0 | 0 | 0 | 0 | 0 | 0 | 0 | 0 | 0 |
| Cough | rmal | 2 | 2 | 1 | 2 | 1 | 2 | 1 | 2 | 1 | 2 | 2 | 2 | 2 | 1 |
| | Intermittence | 0 | 0 | 1 | 0 | 1 | 0 | 1 | 0 | 1 | 0 | 0 | 0 | 0 | 1 |
| | Severe | 0 | 0 | 0 | 0 | 0 | 0 | 0 | 0 | 0 | 0 | 0 | 0 | 0 | 0 |
| Skin rashes | Normal | 2 | 2 | 2 | 2 | 2 | 2 | 2 | 2 | 2 | 2 | 2 | 2 | 2 | 2 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 2 | 2 | 2 | 2 | 2 | 2 | 2 | 2 | 2 | 2 | 2 | 2 | 2 | 2 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |
| Diarrhea | Normal | 1 | 1 | 2 | 2 | 2 | 1 | 1 | 1 | 1 | 1 | 1 | 2 | 2 | 2 |
| | Watery diarrhea | 1 | 1 | 0 | 0 | 0 | 1 | 1 | 1 | 1 | 1 | 1 | 0 | 0 | 0 |
| | Diarrhea | 0 | 0 | 0 | 0 | 0 | 0 | 0 | 0 | 0 | 0 | 0 | 0 | 0 | 0 |

Table 8

| | | No. of individual having clinical symptoms | | | | | | | | | | | | | |
|---|---|---|---|---|---|---|---|---|---|---|---|---|---|---|---|
| | DPI | 1 | 2 | 3 | 4 | 5 | 6 | 7 | 8 | 9 | 10 | 11 | 12 | 13 | 14 |
| Fever | Normal | 1 | 1 | 3 | 1 | 3 | 3 | 2 | 4 | 3 | 4 | 3 | 4 | 4 | 4 |
| | Depressed | 2 | 2 | 0 | 1 | 1 | 0 | 1 | 0 | 0 | 0 | 0 | 0 | 0 | 0 |
| | High fever | 1 | 1 | 1 | 2 | 0 | 1 | 1 | 0 | 1 | 0 | 1 | 0 | 0 | 0 |

(continued)

| | | No. of individual having clinical symptoms | | | | | | | | | | | | | |
|---|---|---|---|---|---|---|---|---|---|---|---|---|---|---|---|
| | DPI | 1 | 2 | 3 | 4 | 5 | 6 | 7 | 8 | 9 | 10 | 11 | 12 | 13 | 14 |
| Breathing | Normal | 3 | 3 | 3 | 4 | 2 | 3 | 3 | 3 | 4 | 4 | 4 | 4 | 4 | 4 |
| | Increase | 1 | 1 | 1 | 0 | 2 | 1 | 1 | 1 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| Cough | Normal | 4 | 4 | 4 | 4 | 4 | 4 | 4 | 4 | 4 | 4 | 4 | 4 | 4 | 4 |
| | Intermittence | 0 | 0 | 0 | 0 | 0 | 0 | 0 | 0 | 0 | 0 | 0 | 0 | 0 | 0 |
| | Severe | 0 | 0 | 0 | 0 | 0 | 0 | 0 | 0 | 0 | 0 | 0 | 0 | 0 | 0 |
| Skin rashes | Normal | 4 | 4 | 4 | 4 | 4 | 4 | 4 | 4 | 4 | 4 | 4 | 4 | 4 | 4 |
| | pots | 0 | 0 | 0 | 0 | 0 | 0 | 0 | 0 | 0 | 0 | 0 | 0 | 0 | 0 |
| | aundice | 0 | 0 | 0 | 0 | 0 | 0 | 0 | 0 | 0 | 0 | 0 | 0 | 0 | 0 |
| Motility | Normal | 4 | 4 | 4 | 4 | 4 | 4 | 4 | 4 | 4 | 4 | 4 | 4 | 4 | 4 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |
| Diarrhea | Normal | 3 | 2 | 4 | 3 | 3 | 4 | 3 | 4 | 4 | 3 | 4 | 3 | 3 | 4 |
| | Watery diarrhea | 1 | 2 | 0 | 1 | 1 | 0 | 1 | 0 | 0 | 1 | 0 | 1 | 1 | 0 |
| | Diarrhea | 0 | 0 | 0 | 0 | 0 | 0 | 0 | 0 | 0 | 0 | 0 | 0 | 0 | 0 |

Table 8 shows clinical symptoms of the second experimental group into which PCV2 and PRRSV were Challenge-inoculated (vaccinated group) after the challenge-inoculation.

Table 9

| | | No. of individual having clinical symptoms | | | | | | | | | | | | | |
|---|---|---|---|---|---|---|---|---|---|---|---|---|---|---|---|
| | DPI | 1 | 2 | 3 | 4 | 5 | 6 | 7 | 8 | 9 | 10 | 11 | 12 | 13 | 14 |
| Fever | Normal | 1 | 1 | 1 | 1 | 2 | 2 | 2 | 2 | 2 | 2 | 2 | 1 | 1 | 1 |
| | Depressed | 1 | 0 | 1 | 1 | 0 | 0 | 0 | 0 | 0 | 0 | 0 | 0 | 0 | 0 |
| | High fever | 0 | 0 | 0 | 0 | 0 | 0 | 0 | 0 | 0 | 0 | 0 | 0 | 0 | 0 |
| Breathing | Normal | 2 | 2 | 2 | 2 | 2 | 1 | 1 | 0 | 0 | 0 | 0 | 0 | 0 | 0 |
| | Increase | 0 | 0 | 0 | 0 | 0 | 1 | 1 | 1 | 1 | 1 | 1 | 0 | 0 | 0 |
| | Opening mouth | 0 | 0 | 0 | 0 | 0 | 0 | 0 | 1 | 1 | 1 | 1 | 1 | 1 | 1 |
| Cough | Normal | 2 | 2 | 2 | 2 | 2 | 2 | 1 | 1 | 0 | 0 | 0 | 0 | 0 | |
| | Intermittence | 0 | 0 | 0 | 0 | 0 | 0 | 1 | 1 | 2 | 2 | 1 | 1 | 1 | 1 |
| | Severe | 0 | 0 | 0 | 0 | 0 | 0 | 0 | 0 | 0 | 0 | 1 | 0 | 0 | 0 |
| Skin rashes | Normal | 2 | 2 | 2 | 2 | 2 | 2 | 2 | 2 | 2 | 2 | 2 | 1 | 1 | 1 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 2 | 2 | 2 | 2 | 2 | 2 | 1 | 1 | 1 | 1 | 1 | 0 | 1 | 1 |
| | Abnormal | 0 | 0 | 0 | 0 | 0 | 0 | 1 | 1 | 1 | 1 | 1 | 1 | 0 | 0 |
| | Daudication | 0 | 0 | 0 | 0 | 0 | 0 | 0 | 0 | 0 | 0 | 0 | 0 | 0 | 0 |

(continued)

| | | No. of individual having clinical symptoms | | | | | | | | | | | | | |
|---|---|---|---|---|---|---|---|---|---|---|---|---|---|---|---|
| | DPI | 1 | 2 | 3 | 4 | 5 | 6 | 7 | 8 | 9 | 10 | 11 | 12 | 13 | 14 |
| Diarrhea | Normal | 2 | 2 | 2 | 2 | 2 | 2 | 1 | 1 | 0 | 0 | 0 | 0 | 0 | 0 |
| | Watery diarrhea | 0 | 0 | 0 | 0 | 0 | 0 | 1 | 1 | 1 | 1 | 1 | 0 | 0 | 0 |
| | Diarrhea | 0 | 0 | 0 | 0 | 0 | 0 | 0 | 0 | 1 | 1 | 1 | 1 | 1 | 1 |

Table 9 shows clinical symptoms of the second control group into which PCV2 and PRRSV were challenge-inoculated (non-vaccinated group) after the challenge-inoculation.

As a result of measuring the elution of the virus via the nasal cavity, it was identified that the PCV2 elution via the nasal cavity was significantly reduced in the first experimental group into which the vaccine was inoculated and the PCV2 was challenge-inoculated and in the second experimental group into which the vaccine was inoculated and PCV2 and PRRSV were challenge-inoculated when compared with the control groups. While the maximum amount of the eluted PCV2 was $10^{0.12}$ $TCID_{50}$/0.1 Me per day in the first and second experimental groups, the maximum amount of the eluted PCV2 was $10^{0.81}TCID_{50}$/0.1 Mℓ in the control groups. In the first control group inoculated with PCV2 and the second control group inoculated with PCV2 and PRRSV, PCV2 was detected since the 3rd day of inoculation. While the maximum amount of the eluted PCV2 was $10^{1.17}$ $TCID_{50}$/0.1 Mℓ per day in the first control group, the maximum amount of the eluted PCV2 was $10^{1.73}$ $TCID_{50}$/0.1 Mℓ in the second control group. In the no-inoculation control group, PCV2 elution was not identified (FIGS. 2 and 3).

FIG. 2 is a graph illustrating PCV2 elution via nasal cavity after PCV2 challenge-inoculation into young pigs. Referring to FIG. 2, DPI indicates date after post infection, IFA $log_{10}$ of the y axis indicates IFA titer obtained using Ct values measured using real time-PCR.

FIG. 3 is a graph illustrating PCV2 elution via nasal cavity after challenge-inoculation of PCV2 and porcine reproductive and respiratory syndrome virus (PRRSV) into young pigs. In FIG. 3, the x and y axes are defined with reference to FIG. 2.

As a result of measuring the elution of the virus via the rectum, it was identified that the PCV2 elution via the excrement was significantly reduced in the first experimental group into which the vaccine was inoculated and the PCV2 was challenge-inoculated and in the second experimental group into which the vaccine was inoculated and PCV2 and PRRSV were challenge-inoculated when compared with the control groups. While the maximum amount of the eluted PCV2 was $10^{0.42}TCID_{50}$/0.1 Mℓ per day in the first and second experimental groups, the maximum amount of the eluted PCV2 was $10^{1.58}$ $TCID_{50}$/0.1 Mℓ in the no-inoculation control group. In the first control group into which PCV2 was inoculated and the second control group into which PCV2 and PRRSV were inoculated, PCV2 was detected since the 3rd day of the inoculation. While the maximum amount of the eluted PCV2 was $10^{1.95}TCID_{50}$/0.1 Mℓ per day in the first control group, the maximum amount of the eluted PCV2 was $10^{1.44}TCID_{50}$/0.1 Mℓ in the second control group. In the no-inoculation control group, no PCV2 elution was identified (FIGS. 4 and 5).

FIG. 4 is a graph illustrating PCV2 elution via excrement after PCV2 challenge-inoculation into young pigs. In FIG. 4, the x and y axes are defined with reference to FIG. 2.

FIG. 5 is a graph illustrating PCV2 elution via excrement after challenge-inoculation of PCV2 and PRRSV into young pigs. In FIG. 5, the x and y axes are defined with reference to FIG. 2.

As described above, in order to identify effectiveness of the vaccine including the attenuated porcine circovirus, pathogenicity and defense capability tests were conducted using pigs. As a result, it was observed that the vaccinated and challenge-inoculated groups did not show particular clinical symptoms compared with the non-vaccinated and challenge-inoculated groups. In addition, it was identified that the elution time of the virus via the nasal cavity or excrement of the vaccinated groups was shorter than that of the control group, and the amount of the eluted virus of the vaccinated groups was significantly less than that of the non-vaccinated groups. Accordingly, it was identified that the vaccine provides excellent defense capability in pigs.

### 5. Outdoor Clinical Trials and Results

#### (5.1) Outdoor Clinical Trials

Vaccine including the attenuated porcine circovirus prepared according to an embodiment of the present invention was administered to pigs raised in outdoor farms to identify safety and effectiveness of the vaccine.

The vaccine was prepared as described above, and three outdoor farms were used for the trials. A first farm raised about 500 sows pigs, a second farm raised about 200 sows, and a third farm raised about 200 sows. The geographical locations of the three farms were different. Table 10 shows the number of pigs used for the trials.

Table 10

| Group | Farm Name | Young Pigs | |
|---|---|---|---|
| | | Inoculation | Control |
| First group | Eden | 20/30a | 20/20 |
| 2nd group | Irae | 20/20 | 20/20 |
| 3rd group | Garam | 20/20 | 20/20 |
| Total | | 120 / 130 | |

a: Number of pigs, blood of which is collected/number of pigs presented.

1 Mℓ of the vaccine was intramuscularly inoculated into each of 3 to 4-week old pigs (inoculation group). After about 2 weeks, the second inoculation of the vaccine was conducted in the same manner. Blood samples of the young pigs were collected before the vaccination (3-week old), after two weeks from the second inoculation (7-week old), after five weeks from the second inoculation (10-week old), after eight weeks from the second inoculation (13-week old), and after 11 weeks from the second inoculation (16-week old), and serum was isolated from their blood.
Clinical symptoms of the young pigs were observed as follows. First, topical and systematic symptoms were observed right after the inoculation of the vaccine.
During the trials, clinical symptoms of the vaccinated group and the control group were observed periodically according to the blood collection. Mainly, cough, appetite disorder, sneeze, mange, dyspnea, vomiting, high fever, bleeding (nose and mouth), lack of strength, general prostration, diarrhea, and the like were observed and the results were recorded. In order to identify outdoor effectiveness and safety of the vaccine, body temperature, weight, and antibody titer of the pigs were measured after the vaccination. The body temperatures of the pigs were measured three times a day (morning, lunch, and dinner) before the inoculation, after 4 hours from the inoculation, and the first and second days from the inoculation, and once a day from the 3rd day to 7th day. The weights of the pigs were measured at the ages of 3 weeks, 10 weeks, 13 weeks, and 16 weeks.
The PCV2 antibody titers were measured at the ages of 3 weeks, 7 weeks, 10 weeks, 13 weeks, and 16 weeks. As the antibody titer of the all pigs, blood of which was collected, PCV2 competitive ELISA antibody titer (Synbiotics) was measured. The PCV2 competitive ELISA antibody titer will be described below.

**PCV2 Ab Mono Blocking (PCV2 competitive ELISA):**

First, controls and samples were prepared. A vial was shaken before use, 100 μℓ of a negative control were added to A1 and A2 wells and 100 μℓ of a positive control were added to B1 and B2 wells. Then, the samples were diluted using a sample-diluting solution to a ratio of 1 : 100, and 100 μℓ of the samples was added to each well. Each sample was placed in one well, or placed in two wells and an average was obtained. The wells were covered with an adhesive film. The plate was cultivated at 37 ± 3 for 1 hour ± 5 minutes. The adhesive film was removed, and then the plate was washed four times with a washing solution diluted to a ratio of 1:10.
A conjugate was diluted to a ratio of 1:100 using a conjugate-diluting solution. 100 μℓ of the diluted conjugate was added to each well and the well was covered with a new adhesive film. The plate was cultivated at 37 ± 3 for 1 hour ± 5 minutes. The adhesive film was removed, and then the plate was washed four times with a washing solution diluted to a ratio of 1:10. Then, 100 μℓ of a substrate solution was added to each well, and the plate was slightly shaken. The plate was cultivated at room temperature for 30 minutes ± 5 minutes while blocking light. Then, 50 μℓ of a stop solution was added to each well, and the plate was slightly shaken. Optical density (OD) was measured at a wavelength of 450 nm.
The results were evaluated according to the following standards. The OD of the control was shown below.

- OD range of the negative control > 0.500
- OD range of the positive control ≤ 0.300

Serum neutralizing antibody titer (SNc) was calculated according to Formula 2 below.

Formula 2

$$SNc = \frac{\text{OD value for the sample} - \text{OD value for the positive control}}{\text{OD value for the negative control} - \text{OD value for the positive control}}$$

If the result is ≤ 0.50, the sample was judged to be positive. If the result is > 0.50, the sample was judged to be negative.

In addition, death rates of the pigs used for the trials were measured, and PCV2 was detected by PCR, immunohistochemistry (IHC), and Salmonella test using PCR.

**(5.2) Results of Outdoor Clinical Trials**

**(5.2.1) First Farm**

The body temperatures of the vaccine-inoculated group and the control group were measured every day before the inoculation and for 7 days after the inoculation. As a result, all individual pigs of the inoculation group and the control group had normal body temperatures for 7 days.

Weights of the inoculation group and the control group were measured at the ages of 3 weeks (before the vaccine inoculation), 10 weeks, 13 weeks, and 16 weeks. Before the vaccine inoculation, the weight of the inoculation group was slightly less than that of the control group. However, at the age of 10 weeks, the weights of the inoculation group and the control group were similar to each other, and at the ages of 13 weeks and 16 weeks, the average weight of the inoculation group was slightly greater than that of the control group. Table 11 shows weights of the inoculation group and the control group (kg, n=20).

Table 11

| | Before inoculation, 3-week old | 10-week old | 13-week old | 16-week old |
|---|---|---|---|---|
| Vaccine Inoculation group | 8.57 | 20.40 | 31.29 | 52.19 |
| Control group | 9.06 | 18.82 | 28.44 | 47.19 |

Blood samples of the pigs of the inoculation group and the control group were collected before the vaccine inoculation (3-week old), and at the ages of 7 weeks, 10 weeks, 13 weeks, and 16 weeks, and antibody titer of PCV2 was measured using ELISA and SN antibody titer was measured using IFA. In the inoculation group, an antibody positive rate significantly increased after two weeks from the second inoculation (7-week old) and the increased antibody positive rate was maintained until the age of 16 weeks. In the control group, the antibody positive rate increased or decreased during the trials and maintained to be lower than that of the inoculation group. The SN antibody titer of the control group was greater than that of the inoculation group before the inoculation (3-week old). However, at the age of 7 weeks, i.e., two weeks from the second inoculation, the SN antibody titer of the inoculation group was significantly greater than that of the control group ($p<0.05$). Table 12 shows PCV2 antibody titers of the inoculation group of the first farm and Table 13 shows PCV2 antibody titers of the control group of the first farm.

Table 12

| No. | Before inoculation 3-week old | | 7-week old | | 10-week old | | 13-week old | | 16 -week old | |
|---|---|---|---|---|---|---|---|---|---|---|
| | ELISA | SN | ELISA | SN | ELISA | SN | ELISA | SN | ELISA | SN |
| 1 | 0.39 | 8 | 0.16 | 64 | 0.22 | 32 | 0.13 | 64 | 0.33 | 16 |
| 2 | 0.33 | 16 | 0.19 | 64 | 0.20 | 32 | 0.21 | 43 | 0.21 | 32 |
| 3 | 0.41 | 4 | 0.14 | 64 | 0.32 | 16 | 0.13 | 64 | 0.21 | 32 |
| 4 | 0.29 | 16 | 0.11 | 64 | 0.21 | 32 | 0.16 | 64 | 0.13 | 64 |
| 5 | 0.39 | 4 | 0.13 | 64 | 0.21 | 32 | 0.22 | 32 | 0.23 | 32 |

(continued)

| | | | | | | | | | | |
|---|---|---|---|---|---|---|---|---|---|---|
| 6 | 0.35 | 8 | 0.20 | 64 | 0.13 | 64 | 0.29 | 16 | 0.26 | 32 |
| 7 | 0.39 | 2 | 0.32 | 16 | 0.16 | 64 | 0.22 | 32 | 0.22 | 32 |
| 8 | 0.31 | 16 | 0.08 | 128 | 0.22 | 32 | 0.20 | 32 | 0.29 | 16 |
| 9 | 0.25 | 16 | 0.16 | 64 | 0.20 | 32 | 0.32 | 16 | 0.24 | 16 |
| 10 | 0.29 | 16 | 0.22 | 32 | 0.28 | 16 | 0.21 | 32 | 0.26 | 16 |
| 11 | 0.30 | 16 | 0.20 | 32 | 0.22 | 32 | 0.20 | 32 | 0.36 | 8 |
| 12 | 0.33 | 16 | 0.32 | 16 | 0.26 | 32 | 0.19 | 32 | 0.23 | 32 |
| 13 | 0.31 | 16 | 0.21 | 32 | 0.19 | 32 | 0.25 | 32 | 0.31 | 32 |
| 14 | 0.40 | 4 | 0.11 | 64 | 0.13 | 64 | 0.22 | 32 | 0.21 | 16 |
| 15 | 0.41 | 2 | 0.13 | 64 | 0.21 | 32 | 0.20 | 32 | - | - |
| 16 | 0.39 | 4 | 0.16 | 64 | 0.13 | 64 | 0.32 | 16 | 0.35 | 16 |
| 17 | 0.41 | 2 | 0.22 | 32 | 0.16 | 64 | 0.26 | 16 | 0.22 | 32 |
| 18 | 0.29 | 16 | 0.20 | 64 | 0.22 | 32 | 0.28 | 32 | 0.36 | 16 |
| 19 | 0.39 | 4 | 0.32 | 16 | 0.20 | 32 | 0.22 | 32 | 0.30 | 32 |
| 20 | 0.35 | 8 | 0.08 | 64 | 0.18 | 32 | 0.20 | 32 | 0.31 | 16 |
| Average | 0.35 | 9.70 | 0.19 | 53.60 | 0.20 | 38.40 | 0.22 | 34.15 | 0.25 | 24.40 |

Table 13

| No. | Before inoculation, 3-week old | | 7-week old | | 10-week old | | 13-week old | | 16-week old | |
|---|---|---|---|---|---|---|---|---|---|---|
| | ELISA | SN | ELISA | SN | ELISA | SN | ELISA | SN | ELISA | SN |
| 1 | 0.28 | 32 | 0.39 | 8 | 0.36 | 16 | 0.36 | 8 | 0.35 | 8 |
| 2 | 0.25 | 32 | 0.41 | 4 | 0.37 | 16 | 0.37 | 16 | 0.39 | 2 |
| 3 | 0.29 | 16 | 0.29 | 16 | 0.27 | 32 | 0.14 | 64 | 0.30 | 16 |
| 4 | 0.30 | 16 | 0.39 | 8 | 0.16 | 64 | 0.32 | 16 | 0.23 | 32 |
| 5 | 0.33 | 16 | 0.35 | 8 | 0.31 | 32 | 0.32 | 16 | 0.33 | 16 |
| 6 | 0.31 | 16 | 0.36 | 8 | 0.25 | 32 | 0.31 | 32 | 0.24 | 32 |
| 7 | 0.36 | 16 | 0.35 | 8 | 0.29 | 16 | 0.33 | 16 | 0.35 | 8 |
| 8 | 0.37 | 8 | 0.39 | 4 | 0.26 | 32 | 0.31 | 16 | 0.36 | 8 |
| 9 | 0.27 | 32 | 0.31 | 16 | 0.33 | 16 | 0.35 | 8 | 0.25 | 32 |
| 10 | 0.21 | 32 | 0.25 | 32 | 0.34 | 16 | 0.28 | 16 | 0.30 | 16 |
| 11 | 0.32 | 16 | 0.29 | 16 | 0.28 | 32 | 0.22 | 32 | 0.36 | 8 |
| 12 | 0.41 | 4 | 0.30 | 16 | 0.21 | 32 | 0.26 | 16 | 0.37 | 8 |
| 13 | 0.38 | 8 | - | - | - | - | - | - | - | - |
| 14 | 0.26 | 32 | 0.23 | 32 | 0.20 | 32 | 0.36 | 8 | 0.44 | 0 |
| 15 | 0.37 | 8 | 0.31 | 16 | 0.16 | 64 | 0.40 | 4 | 0.29 | 16 |
| 16 | 0.27 | 32 | 0.25 | 32 | 0.37 | 16 | 0.24 | 32 | 0.41 | 2 |
| 17 | 0.21 | 32 | 0.29 | 32 | 0.34 | 16 | 0.30 | 16 | 0.33 | 16 |
| 18 | 0.32 | 16 | 0.26 | 32 | 0.25 | 32 | 0.38 | 8 | 0.31 | 32 |

(continued)

| No. | Before inoculation, 3-week old | | 7-week old | | 10-week old | | 13-week old | | 16-week old | |
|---|---|---|---|---|---|---|---|---|---|---|
| | ELISA | SN | ELISA | SN | ELISA | SN | ELISA | SN | ELISA | SN |
| 19 | 0.30 | 16 | 0.33 | 16 | 0.26 | 16 | 0.33 | 16 | 0.20 | 32 |
| 20 | 0.38 | 8 | 0.31 | 16 | 0.20 | 32 | 0.34 | 16 | 0.31 | 16 |
| Average | 0.31 | 19.40 | 0.30 | 16.00 | 0.26 | 27.20 | 0.29 | 17.80 | 0.31 | 15.00 |

In the inoculation group, no pig died for 16 weeks after the vaccine was inoculated. In the control group, one pig died at the age of 7 weeks. Lymph nodes and lung were positive to PCV2 as a result of conducting PCR using a tissue sample of the dead pig of the control group as a template but negative to PCV2 in the tissue IHC staining.

During the trials, a Salmonella test was conducted. The dead pig was negative to Salmonella.

Clinical symptoms of the inoculation group and the control group were observed by the age of 16 weeks. While most of the pigs of the inoculation group showed normal conditions, some of the pigs of the control group showed PCV2 infection-associated symptoms such as depression, Watery diarrhea, rough hair, and atrophy. Table 14 shows clinical symptoms of the inoculation group, and Table 15 shows clinical symptoms of the control group.

Table 14

| | | No. of pigs | | | | | | | | | | | | | |
|---|---|---|---|---|---|---|---|---|---|---|---|---|---|---|---|
| | week | 3 | 4 | 5 | 6 | 7 | 8 | 9 | 10 | 11 | 12 | 13 | 14 | 15 | 16 |
| Breathing | Normal | 30 | 30 | 30 | 30 | 30 | 30 | 30 | 30 | 30 | 30 | 30 | 30 | 30 | 30 |
| | Increase | 0 | 0 | 0 | 0 | 0 | 0 | 0 | 0 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| Cough | Normal | 27 | 27 | 28 | 28 | 29 | 30 | 30 | 30 | 30 | 30 | 30 | 30 | 30 | 30 |
| | Intermittence | 3 | 3 | 2 | 2 | 1 | 0 | 0 | 0 | 0 | 0 | 0 | 0 | 0 | 0 |
| | Severe | 0 | 0 | 0 | 0 | 0 | 0 | 0 | 0 | 0 | 0 | 0 | 0 | 0 | 0 |
| Skin rashes | Normal | 30 | 30 | 30 | 30 | 30 | 30 | 30 | 30 | 30 | 30 | 30 | 30 | 30 | 30 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 30 | 30 | 30 | 30 | 30 | 30 | 30 | 30 | 30 | 30 | 30 | 30 | 30 | 30 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |
| Diarrhea | Normal | 30 | 30 | 30 | 30 | 29 | 29 | 30 | 30 | 30 | 30 | 29 | 29 | 30 | 30 |
| | Watery diarrhea | 0 | 0 | 0 | 0 | 1 | 1 | 0 | 0 | 0 | 0 | 1 | 1 | 0 | 0 0 |
| | Diarrhea | 0 | 0 | 0 | 0 | 0 | 0 | 0 | 0 | 0 | 0 | 0 | 0 | 0 | 0 |

Table 15

| | | No. of pigs | | | | | | | | | | | | | |
|---|---|---|---|---|---|---|---|---|---|---|---|---|---|---|---|
| | week | 3 | 4 | 5 | 6 | 7 | 8 | 9 | 10 | 11 | 12 | 13 | 14 | 15 | 16 |
| Breathing | Normal | 20 | 20 | 20 | 18 | 19 | 19 | 19 | 19 | 19 | 19 | 19 | 19 | 19 | 19 |
| | Increase | 0 | 0 | 0 | 2 | 0 | 0 | 0 | 0 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| Cough | Normal | 20 | 20 | 20 | 18 | 18 | 19 | 19 | 18 | 17 | 17 | 19 | 19 | 19 | 19 |
| | Intermittence | 0 | 0 | 0 | 2 | 1 | 0 | 0 | 1 | 2 | 2 | 0 | 0 | 0 | 0 |
| | Severe | 0 | 0 | 0 | 0 | 0 | 0 | 0 | 0 | 0 | 0 | 0 | 0 | 0 | 0 |
| Skin rashes | Normal | 20 | 20 | 20 | 20 | 19 | 19 | 19 | 19 | 19 | 19 | 19 | 19 | 19 | 19 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 20 | 20 | 20 | 20 | 19 | 19 | 19 | 19 | 19 | 19 | 19 | 19 | 19 | 19 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |
| Diarrhea | Normal | 20 | 20 | 20 | 20 | 19 | 19 | 17 | 17 | 18 | 17 | 19 | 19 | 17 | 17 |
| | Watery diarrhea | 0 | 0 | 0 | 0 | 0 | 0 | 2 | 2 | 1 | 2 | 0 | 0 | 2 | 2 |
| | Diarrhea | 0 | 0 | 0 | 0 | 0 | 0 | 0 | 0 | 0 | 0 | 0 | 0 | 0 | 0 |

**(5.2.2) Second Farm**

The body temperatures of the vaccine-inoculated group and the control group were measured every day before the inoculation and for 7 days after the inoculation. All individual pigs of the inoculation group and the control group showed normal body temperatures for 7 days.

Weights of the inoculation group and the control group were measured at the ages of 3 weeks (before the vaccine inoculation), 10 weeks, 13 weeks, and 16 weeks. Until the age of 10 weeks, the weights of the inoculation group and the control group were similar to each other, and at the ages of 13 weeks and 16 weeks, the average weight of the inoculation group was slightly greater than that of the control group. Table 16 shows weights of the inoculation group and the control group (kg, n=20).

Table 16

| | Before inoculation, 3-week old | 10-week old | 13-week old | 16-week old |
|---|---|---|---|---|
| Vaccine inoculation group | 8.57 | 20.04 | 31.93 | 51.82 |
| Control group | 8.79 | 19.15 | 29.08 | 49.32 |

Blood samples of the pigs of the inoculation group and the control group were collected before the vaccine inoculation (3-week old), and at the ages of 10 weeks, 13 weeks, and 16 weeks, and antibody titer of PCV2 was measured using ELISA and SN antibody titer was measured using IFA. In the inoculation group, an antibody titer was significantly increased after two weeks from the second inoculation (7-week old) and the increased antibody titer maintained until the age of 16 weeks. In the control group, the antibody positive rate and the antibody titer gradually increased to be similar to those of the inoculation group at the age of 13 weeks. The SN antibody titers of the inoculation group and the control group were seronegative (≤ 1: 10). However, after two weeks from the second inoculation (7-week old), an average SN antibody titer of the inoculation group was 1: 25, and an average SN antibody titer of the control group was 1:10 to exhibit a significant difference ($p<0.05$), and the difference increased until the age of 10 weeks. Since the antibody titer of the control group increased from the age of 13 weeks so as to be similar to that of the inoculation group. Table 17 shows PCV2 antibody titer of the inoculation group of the second farm and Table 18 shows PCV2 antibody titer of the control group of the second farm.

Table 17

| No. | Before inoculation, 3-week old | | 7-week old | | 10-week old | | 13-week old | | 16-week old | |
|---|---|---|---|---|---|---|---|---|---|---|
| | ELISA | SN | ELISA | SN | ELISA | SN | ELISA | SN | ELISA | SN |
| 1 | 0.48 | 4 | 0.34 | 4 | 0.39 | 4 | 0.23 | 16 | 0.15 | 64 |
| 2 | 0.49 | 4 | 0.25 | 16 | 0.25 | 16 | 0.29 | 16 | 0.28 | 16 |
| 3 | 0-38 | 4 | 0.22 | 32 | 0.11 | 64 | 0.20 | 32 | 0.31 | 16 |
| 4 | 0.55 | 0 | 0.31 | 16 | 0.15 | 64 | 0.13 | 64 | 0.28 | 16 |
| 5 | 0.45 | 2 | 0.39 | 4 | 0.15 | 64 | 0.17 | 64 | 0.10 | 128 |
| 6 | 0.56 | 2 | 0.27 | 32 | 0.26 | 32 | 0.20 | 32 | 0.14 | 64 |
| 7 | 0.56 | 0 | 0.15 | 128 | 0.07 | 128 | 0.08 | 128 | 0.09 | 128 |
| 8 | 0.39 | 4 | 0.25 | 32 | 0.18 | 64 | 0.29 | 16 | 0.28 | 16 |
| 9 | 0.45 | 4 | 0.38 | 8 | 0.11 | 64 | 0.13 | 32 | 0.12 | 64 |
| 10 | 0.35 | 8 | 0.31 | 16 | 0.08 | 64 | 0.10 | 64 | 0.09 | 64 |
| 11 | 0.30 | 16 | 0.19 | 64 | 0.20 | 32 | 0.2 | 16 | 0.13 | 64 |
| 12 | 0.49 | 4 | 0.28 | 32 | 0.34 | 4 | 0.24 | 16 | 0.26 | 32 |
| 13 | 0.23 | 32 | 0.30 | 16 | 0.11 | 64 | 0.10 | 64 | 0.15 | 64 |
| 14 | 0.26 | 32 | 0.16 | 64 | 0.23 | 16 | 0.14 | 64 | 0.20 | 32 |
| 15 | 0.34 | 8 | 0.40 | 2 | 0.14 | 32 | 0.28 | 16 | 0.30 | 16 |
| 16 | 0.38 | 4 | 0.39 | 4 | 0.18 | 64 | 0.17 | 64 | 0.14 | 64 |
| 17 | 0.46 | 2 | 0.34 | 4 | 0.15 | 64 | 0.19 | 32 | 0.20 | 32 |
| 18 | 0.50 | 2 | 0.25 | 16 | 0.29 | 16 | 0.22 | 16 | 0.20 | 64 |
| 19 | 0.41 | 2 | 0.48 | 2 | 0.37 | 8 | 0.21 | 32 | 0.18 | 64 |
| 20 | 0.50 | 2 | 0.26 | 16 | 0.17 | 64 | 0.25 | 16 | 0.19 | 64 |
| Average | 0.42 | 6.80 | 0.30 | 25.40 | 0.20 | 46.40 | 0.19 | 40.00 | 0.19 | 53.60 |

Table 18

| No. | Before inoculation 3-week old | | 7-week old | | 10-week old | | 13-week old | | 16 -week old | |
|---|---|---|---|---|---|---|---|---|---|---|
| | ELISA | SN | ELISA | SN | ELISA | SN | ELISA | SN | ELISA | SN |
| 1 | 0.42 | 2 | 0.35 | 4 | 0.45 | 2 | 0.28 | 16 | 0.29 | 16 |
| 2 | 0.53 | 0 | 0.50 | 0 | 0.51 | 0 | 0.15 | 64 | 0.18 | 64 |
| 3 | 0.45 | 0 | 0.49 | 0 | 0.44 | 0 | 0.29 | 16 | 0.25 | 32 |
| 4 | 0.55 | 0 | 0.41 | 2 | 0.41 | 2 | 0.42 | 2 | 0.30 | 16 |
| 5 | 0.48 | 0 | 0.39 | 4 | 0.38 | 4 | 0.18 | 64 | 0.19 | 64 |
| 6 | 0.25 | 16 | 0.45 | 2 | 0.37 | 4 | 0.31 | 8 | 0.18 | 64 |
| 7 | 0.34 | 8 | 0.18 | 64 | 0.19 | 32 | 0.12 | 64 | 0.16 | 64 |
| 8 | 0.21 | 32 | 0.29 | 8 | 0.28 | 16 | 0.08 | 64 | 0.14 | 64 |
| 9 | 0.28 | 16 | 0.24 | 16 | 0.25 | 32 | 0.19 | 32 | 0.13 | 64 |

(continued)

| No. | Before inoculation 3-week old | | 7-week old | | 10-week old | | 13-week old | | 16 -week old | |
|---|---|---|---|---|---|---|---|---|---|---|
| | ELISA | SN | ELISA | SN | ELISA | SN | ELISA | SN | ELISA | SN |
| 10 | 0.30 | 8 | 0.37 | 4 | 0.39 | 4 | 0.20 | 32 | 0.21 | 32 |
| 11 | 0.52 | 0 | 0.49 | 0 | 0.39 | 2 | 0.20 | 32 | 0.18 | 64 |
| 12 | 0.45 | 2 | 0.42 | 2 | 0.14 | 64 | 0.13 | 64 | 0.15 | 64 |
| 13 | 0.40 | 2 | 0.32 | 4 | 0.38 | 4 | 0.15 | 64 | 0.19 | 32 |
| 14 | 0.39 | 4 | 0.34 | 4 | 0.37 | 4 | 0.18 | 64 | 0.20 | 32 |
| 15 | 0.47 | 0 | 0.34 | 8 | 0.39 | 4 | 0.16 | 64 | 0.17 | 64 |
| 16 | 0.29 | 8 | 0.37 | 4 | 0.26 | 16 | 0.20 | 32 | 0.18 | 64 |
| 17 | 0.13 | 64 | 0.17 | 64 | 0.12 | 64 | 0.26 | 32 | 0.21 | 32 |
| 18 | 0.43 | 2 | 0.43 | 2 | 0.45 | 2 | 0.22 | 32 | 0.27 | 16 |
| 19 | 0.48 | 2 | 0.28 | 16 | 0.29 | 16 | 0.20 | 32 | 0.28 | 16 |
| 20 | 0.49 | 0 | 0.49 | 0 | 0.47 | 0 | 0.12 | 64 | 0.06 | 128 |
| Average | 0.39 | 8.30 | 0.36 | 10.40 | 0.34 | 13.60 | 0.21 | 42.10 | 0.20 | 49.60 |

No pig died from the inoculation group and the control group.

Clinical symptoms of the inoculation group and the control group were observed by the age of 16 weeks. While most of the pigs of the inoculation group showed normal conditions, about three pigs of the control group showed watery diarrhea and rough hair. Table 19 shows clinical symptoms of the inoculation group, and Table 20 shows clinical symptoms of the control group.

Table 19

| | | No. of individuals having clinical symptoms | | | | | | | | | | | | | |
|---|---|---|---|---|---|---|---|---|---|---|---|---|---|---|---|
| | week | 3 | 4 | 5 | 6 | 7 | 8 | 9 | 10 | 11 | 12 | 13 | 14 | 15 | 16 |
| Breathin g | Normal | 20 | 20 | 20 | 20 | 20 | 20 | 20 | 20 | 20 | 20 | 20 | 20 | 20 | 20 |
| | ncrease | 0 | 0 | 0 | 0 | 0 | 0 | 0 | 0 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| Cough | Normal | 20 | 20 | 20 | 20 | 20 | 20 | 20 | 20 | 20 | 20 | 20 | 20 | 20 | 20 |
| | Intermittence | 0 | 0 | 0 | 0 | 0 | 0 | 0 | 0 | 0 | 0 | 0 | 0 | 0 | 0 |
| | Severe | 0 | 0 | 0 | 0 | 0 | 0 | 0 | 0 | 0 | 0 | 0 | 0 | 0 | 0 |
| Skin rashes | Normal | 20 | 20 | 20 | 20 | 20 | 20 | 20 | 20 | 20 | 20 | 20 | 20 | 20 | 20 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal Abnormal | 200 | 2200 | 200 | 200 | 200 | 200 | 200 | 200 | 200 | 200 | 200 | 200 | 200 | 200 |
| | udication | 0 | 0 | 0 | 0 | 0 | 0 | 0 | 0 | 0 | 0 | 0 | 0 | 0 | 0 |
| Diarrhea | Normal | 20 | 20 | 20 | 20 | 20 | 20 | 20 | 20 | 20 | 20 | 20 | 20 | 20 | 20 |
| | Watery diarrhea | 0 | 0 | 0 | 0 | 0 | 0 | 0 | 0 | 0 | 0 | 0 | 0 | 0 | 0 |
| | Diarrhea | 0 | 0 | 0 | 0 | 0 | 0 | 0 | 0 | 0 | 0 | 0 | 0 | 0 | 0 |

Table 20

| | | No. of individuals having clinical symptoms | | | | | | | | | | | | | |
|---|---|---|---|---|---|---|---|---|---|---|---|---|---|---|---|
| | week | 3 | 4 | 5 | 6 | 7 | 8 | 9 | 10 | 11 | 12 | 13 | 14 | 15 | 16 |
| Breathing | Normal | 20 | 20 | 20 | 20 | 20 | 20 | 20 | 20 | 20 | 20 | 20 | 20 | 20 | 20 |
| | Increase | 0 | 0 | 0 | 0 | 0 | 0 | 0 | 0 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| Cough | Normal | 20 | 20 | 20 | 20 | 20 | 20 | 20 | 20 | 20 | 20 | 20 | 20 | 20 | 20 |
| | Intermittence | 0 | 0 | 0 | 0 | 0 | 0 | 0 | 0 | 0 | 0 | 0 | 0 | 0 | 0 |
| | Severe | 0 | 0 | 0 | 0 | 0 | 0 | 0 | 0 | 0 | 0 | 0 | 0 | 0 | 0 |
| Skin rashes | Normal | 20 | 20 | 20 | 20 | 20 | 20 | 20 | 20 | 20 | 20 | 20 | 20 | 20 | 20 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 20 | 20 | 20 | 20 | 20 | 20 | 20 | 20 | 20 | 20 | 20 | 20 | 20 | 20 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |
| iarrhea | Normal | 20 | 20 | 20 | 20 | 19 | 19 | 19 | 20 | 20 | 20 | 20 | 20 | 20 | 20 |
| | Watery diarrhea | 0 | 0 | 0 | 0 | 1 | 1 | 1 | 0 | 0 | 0 | 0 | 0 | 0 | 0 |
| | Diarrhea | 0 | 0 | 0 | 0 | 0 | 0 | 0 | 0 | 0 | 0 | 0 | 0 | 0 | 0 |

**(5.2.3) Third Farm**

Body temperatures of the vaccine-inoculated group and the control group were measured every day before the inoculation and for 7 days after the inoculation. All individual pigs of the inoculation group and the control group had normal body temperatures for 7 days.

Weights of the inoculation group and the control group were measured at the ages of 3 weeks (before the vaccine inoculation), 10 weeks, 13 weeks, and 16 weeks. After the age of 10 weeks, an average weight of the pigs in the inoculation group was greater than that of the control group. Table 21 shows weights of the inoculation group and the control group (kg, n=20).

Table 21

| | Before inoculation, 3-week old | 10-week old | 13-week old | 16-week old |
|---|---|---|---|---|
| Vaccine inoculation group | 4.89 | 27.66 | 40.16 | 53.26 |
| Control group | 4.90 | 25.62 | 36.81 | 47.91 |

Blood samples of the pigs of the inoculation group and the control group were collected before the vaccine inoculation (3-week old), and at the ages of 10 weeks, 13 weeks, and 16 weeks, and antibody titer of PCV2 was measured using ELISA and SN antibody titer was measured using IFA. In the inoculation group, an antibody positive rate and an antibody titer was significantly increased after two weeks from the second inoculation (7-week old) and the increased antibody positive rate and antibody titer maintained until the age of 16 weeks. In the control group, the antibody positive rate gradually increased. However, a negative individual was identified at the age of 16 weeks. The SN antibody titers of the inoculation group and the control group were seronegative (≤ 1: 10). However, after the second inoculation, an average SN antibody titer of the inoculation group was 1: 33), and an average SN antibody titer of the control group was 1: 15 to exhibit a significant difference ($p<0.05$). Then, this significant difference was maintained until the age of 16 weeks. Table 22 shows PCV2 antibody titer of the inoculation group of the third farm and Table 23 shows PCV2 antibody titer of the control group of the third farm.

Table 22

| No. | Before inoculation 3-week old | | 7-week old | | 10-week old | | 13-week old | | 16-week old | |
|---|---|---|---|---|---|---|---|---|---|---|
| | ELISA | SN | ELISA | SN | ELISA | SN | ELISA | SN | ELISA | SN |
| 1 | 0.25 | 32 | 0.35 | 16 | 0.28 | 32 | 0.25 | 32 | 0.20 | 64 |
| 2 | 0.40 | 4 | 0.42 | 2 | 0.26 | 32 | 0.28 | 32 | 0.21 | 32 |
| 3 | 0.49 | 0 | 0.28 | 32 | 0.27 | 32 | 0.21 | 64 | 0.24 | 32 |
| 4 | 0.53 | 0 | 0.19 | 64 | 0.14 | 64 | 0.14 | 64 | 0.16 | 32 |
| 5 | 0.47 | 0 | 0.27 | 32 | 0.19 | 64 | 0.15 | 64 | 0.26 | 16 |
| 6 | 0.41 | 2 | 0.40 | 2 | 0.35 | 16 | 0.29 | 16 | 0.23 | 32 |
| 7 | 0.51 | 0 | 0.15 | 64 | 0.14 | 64 | 0.13 | 64 | 0.21 | 32 |
| 8 | 0.47 | 0 | 0.23 | 32 | 0.26 | 32 | 0.28 | 32 | 0.20 | 32 |
| 9 | 0.49 | 0 | 0.24 | 32 | 0.21 | 32 | 0.23 | 32 | 0.27 | 32 |
| 10 | 0.52 | 0 | 0.18 | 64 | 0.17 | 64 | 0.19 | 64 | 0.16 | 64 |
| 11 | 0.45 | 2 | 0.38 | 8 | 0.28 | 32 | 0.21 | 64 | 0.26 | 32 |
| 12 | 0.42 | 2 | 0.47 | 0 | 0.46 | 2 | 0.31 | 16 | 0.20 | 32 |
| 13 | 0.28 | 16 | 0.29 | 16 | 0.24 | 32 | 0.18 | 6 | 0.21 | 64 |
| 14 | 0.46 | 0 | 0.40 | 2 | 0.42 | 2 | 0.36 | 16 | 0.26 | 32 |
| 15 | 0.50 | 0 | 0.27 | 32 | 0.29 | 16 | 0.24 | 32 | 0.25 | 32 |
| 16 | 0.54 | 0 | 0.14 | 64 | 0.13 | 64 | 0.17 | 64 | 0.19 | 64 |
| 17 | 0.38 | 4 | 0.46 | 2 | 0.19 | 32 | 0.11 | 64 | 0.14 | 64 |
| 18 | 0.45 | 2 | 0.24 | 32 | 0.16 | 64 | 0.13 | 64 | 0.18 | 64 |
| 19 | 0.49 | 0 | 0.25 | 32 | 0.21 | 32 | 0.21 | 32 | 0.21 | 32 |
| 20 | 0.52 | 0 | 0.09 | 128 | 0.10 | 64 | 0.12 | 64 | 0.18 | 64 |
| Average | 0.45 | 3.20 | 0.30 | 32.80 | 0.24 | 38.60 | 0.21 | 47.20 | 0.21 | 42.40 |

Table 23

| No. | Before inoculation, 3-week old | | 7-week old | | 10-week old | | 13-week old | | 16-week old | |
|---|---|---|---|---|---|---|---|---|---|---|
| | ELISA | SN | ELISA | SN | ELISA | SN | ELISA | SN | ELISA | SN |
| 1 | 0.51 | 0 | 0.47 | 0 | 0.38 | 8 | 0.39 | 8 | 0.30 | 32 |
| 2 | 0.50 | 0 | 0.54 | 0 | 0.47 | 0 | 0.39 | 8 | 0.31 | 16 |
| 3 | 0.40 | 4 | 0.37 | 8 | 0.34 | 16 | 0.36 | 8 | 0.24 | 32 |
| 4 | 0.54 | 0 | 0.45 | 2 | 0.32 | 16 | 0.31 | 16 | 0.34 | 16 |
| 5 | 0.27 | 32 | 0.32 | 16 | 0.24 | 32 | 0.22 | 32 | 0.21 | 32 |
| 6 | 0.41 | 2 | 0.40 | 4 | 0.21 | 32 | 0.20 | 32 | 0.14 | 64 |
| 7 | 0.41 | 4 | 0.35 | 16 | 0.37 | 16 | 0.39 | 8 | 0.40 | 8 |
| 8 | 0.38 | 8 | 0.34 | 16 | 0.24 | 32 | 0.26 | 32 | 0.19 | 64 |
| 9 | 0.42 | 2 | 0.41 | 4 | 0.45 | 2 | 0.46 | 2 | 0.44 | 2 |
| 10 | 0.26 | 32 | 0.25 | 32 | 0.22 | 32 | 0.19 | 64 | 0.20 | 32 |

(continued)

| No. | Before inoculation, 3-week old | | 7-week old | | 10-week old | | 13-week old | | 16-week old | |
|---|---|---|---|---|---|---|---|---|---|---|
| | ELISA | SN | ELISA | SN | ELISA | SN | ELISA | SN | ELISA | SN |
| 11 | 0.48 | 0 | 0.42 | 4 | 0.44 | 2 | 0.41 | 4 | 0.43 | 2 |
| 12 | 0.53 | 0 | 0.51 | 0 | 0.50 | 0 | 0.51 | 0 | 0.50 | 0 |
| 13 | 0.47 | 0 | 0.48 | 0 | 0.51 | 0 | 0.47 | 0 | 0.49 | 0 |
| 14 | 0.34 | 16 | 0.35 | 16 | 0.32 | 16 | 0.30 | 32 | 0.31 | 16 |
| 15 | 0.47 | 2 | 0.41 | 4 | 0.43 | 2 | 0.24 | 32 | 0.21 | 64 |
| 16 | 0.33 | 16 | 0.34 | 32 | 0.12 | 64 | 0.14 | 64 | 0.12 | 64 |
| 17 | 0.44 | 2 | 0.21 | 64 | 0.24 | 32 | 0.20 | 32 | 0.21 | 64 |
| 18 | 0.51 | 0 | 0.14 | 64 | 0.15 | 64 | 0.21 | 32 | 0.18 | 64 |
| 19 | 0.43 | 2 | 0.37 | 16 | 0.39 | 8 | 0.33 | 32 | 0.34 | 16 |
| 20 | 0.52 | 0 | 0.55 | 0 | 0.47 | 0 | 0.51 | 0 | 0.50 | 0 |
| Average | 0.43 | 6.10 | 0.37 | 14.90 | 0.30 | 18.70 | 0.24 | 21.90 | 0.22 | 29.40 |

During the trials, there was no dead pig in the inoculation group and the control group.

Clinical symptoms of the inoculation group and the control group were observed by the age of 16 weeks. While most of the pigs of the inoculation group and the control group showed normal conditions, some pigs of the control group showed cough and watery diarrhea, but recovered after treatment with antibiotics. Table 24 shows clinical symptoms of the inoculation group, and Table 25 shows clinical symptoms of the control group.

Table 24

| | | No. of individuals having clinical symptoms | | | | | | | | | | | | | |
|---|---|---|---|---|---|---|---|---|---|---|---|---|---|---|---|
| | week | 3 | 4 | 5 | 6 | 7 | 8 | 9 | 10 | 11 | 12 | 13 | 14 | 15 | 16 |
| Breathing | Normal | 20 | 20 | 20 | 20 | 20 | 20 | 20 | 20 | 20 | 20 | 20 | 20 | 20 | 20 |
| | Increase | 0 | 0 | 0 | 0 | 0 | 0 | 0 | 0 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| Cough | Normal | 20 | 20 | 20 | 20 | 20 | 20 | 20 | 20 | 20 | 20 | 20 | 20 | 20 | 20 |
| | Intermittence | 0 | 0 | 0 | 0 | 0 | 0 | 0 | 0 | 0 | 0 | 0 | 0 | 0 | 0 |
| | Severe | 0 | 0 | 0 | 0 | 0 | 0 | 0 | 0 | 0 | 0 | 0 | 0 | 0 | 0 |
| Skin rashes | Normal | 20 | 20 | 20 | 20 | 20 | 20 | 20 | 20 | 20 | 20 | 20 | 20 | 20 | 20 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 20 | 20 | 20 | 20 | 20 | 20 | 20 | 20 | 20 | 20 | 20 | 20 | 20 | 20 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |
| Diarrhea | Normal | 20 | 20 | 20 | 20 | 20 | 20 | 20 | 20 | 20 | 20 | 20 | 20 | 20 | 20 |
| | Watery diarrhea | 0 | 0 | 0 | 0 | 0 | 0 | 0 | 0 | 0 | 0 | 0 | 0 | 0 | 0 |
| | Diarrhea | 0 | 0 | 0 | 0 | 0 | 0 | 0 | 0 | 0 | 0 | 0 | 0 | 0 | 0 |

Table 25

| | | No. of individuals having clinical symptoms | | | | | | | | | | | | | |
|---|---|---|---|---|---|---|---|---|---|---|---|---|---|---|---|
| | week | 3 | 4 | 5 | 6 | 7 | 8 | 9 | 10 | 11 | 12 | 13 | 14 | 15 | 16 |
| Breathing | Normal | 20 | 20 | 20 | 20 | 20 | 20 | 20 | 20 | 20 | 20 | 20 | 20 | 20 | 20 |
| | Increase | 0 | 0 | 0 | 0 | 0 | 0 | 0 | 0 | 0 | 0 | 0 | 0 | 0 | 0 |
| | Opening mouth | 0 | 0 | 0 | 0 | 0 | 0 | 0 | 0 | 0 | 0 | 0 | 0 | 0 | 0 |
| | Normal | 20 | 20 | 19 | 18 | 19 | 20 | 20 | 20 | 20 | 20 | 20 | 20 | 20 | 20 |
| Cough | Intermeittence | 0 | 0 | 1 | 2 | 1 | 0 | 0 | 0 | 0 | 0 | 0 | 0 | 0 | 0 |
| | Severe | 0 | 0 | 0 | 0 | 0 | 0 | 0 | 0 | 0 | 0 | 0 | 0 | 0 | 0 |
| Skin ashes | Normal | 20 | 20 | 20 | 20 | 20 | 20 | 20 | 20 | 20 | 20 | 20 | 20 | 20 | 20 |
| | Spots | 0 | 0 | 0 | 0 | 0 | 0 | 0 | 0 | 0 | 0 | 0 | 0 | 0 | 0 |
| | Jaundice | 0 | 0 | 0 | 0 | 0 | 0 | 0 | 0 | 0 | 0 | 0 | 0 | 0 | 0 |
| Motility | Normal | 20 | 20 | 20 | 20 | 20 | 20 | 20 | 20 | 20 | 20 | 20 | 20 | 20 | 20 |
| | Abnormal | 0 | 0 | 0 | 0 | 0 | 0 | 0 | 0 | 0 | 0 | 0 | 0 | 0 | 0 |
| | Daudication | 0 | 0 | 0 | 0 | 0 | 0 | 0 | 0 | 0 | 0 | 0 | 0 | 0 | 0 |
| Diarrhea | Normal | 20 | 20 | 20 | 20 | 20 | 18 | 19 | 19 | 20 | 20 | 20 | 20 | 20 | 20 |
| | Watery diarrhea | 0 | 0 | 0 | 0 | 0 | 2 | 1 | 1 | 0 | 0 | 0 | 0 | 0 | 0 |
| | Diarrhea | 0 | 0 | 0 | 0 | 0 | 0 | 0 | 0 | 0 | 0 | 0 | 0 | 0 | 0 |

**(5.3) Conclusion**

The outdoor clinical trials were conducted to determine the safety and immunogenicity of the vaccine including the attenuated porcine circovirus according to an embodiment of the present invention for outdoor farms.

130 young pigs from three different outdoor farms were used for the trials, and 1 Mℓ of the immunogenic composition was intramuscularly inoculated twice at the age of 3 weeks and at the age of 5 weeks. As a result of observing clinical symptoms after the inoculation, there was no topical and systematical symptom that is commonly observed after 1 to 2 hours after the inoculation. In addition, clinical symptoms such as dyspnea, vomiting, bleeding, high fever, and general prostration were not observed during the trials. Even though some of the pigs exhibited appetite disorder but recovered within several days.

When the overall antibody titer after the second inoculation was measured from the three outdoor farms, a sero-conversion rate of the antibody against the circovirus was equal to or greater than 90% after the second inoculation compared to the antibody titer before the inoculation, thereby exhibiting excellent immunogenicity.

Natural cumulative death rate was about zero until the age of 16 weeks from the vaccine inoculation in the inoculation group and the control group. The weight gain rate of the vaccinated group was greater than that of the control group, thereby exhibiting a big difference of average weights between the pigs having the same age. According to the results described above, safety and defense capability of the vaccine in outdoor farms were sufficiently proved.

**6. Comparative Experiment**

Effectiveness of the vaccine including the attenuated porcine circovirus according to the present invention was evaluated by comparison with a commercially available product. Ingelvac CircoFlex™ 309-129, 30991-kr2 (Boehringer Ingelhein GmbH, Korea) was used for the comparison. Ingelvac CircoFlex™ includes purified circovirus antigen (PCV™) and an excipient of Impran FLEX™ that is an aqueous polymer.

3-week old young pigs from the first farm were used for the comparative experiment. 1 Mℓ of the immunogenic composition according to the present invention was intramuscularly inoculated into behind the base of the ear of 3-week old pigs and 5-week old pigs to prepare group A, and 1 Mℓ of Ingelvac CircoFlex™ was intramuscularly inoculated into behind the base of the ear of 3-week old pigs to prepare group B. Non-vaccinated control group was prepared as group C (Table 26).

Table 26

| Group | No. of pigs | Vaccine inoculation |
|---|---|---|
| A | 750 | Vaccine of the present invention; intramuscular inoculation, twice at the ages of 3-week and 5-week old |
| B | 98 | Ingelvac CircoFlex, intramuscular inoculation once at the age of 3-week old |
| C | 49 | Non-Vaccinated |

Since Ingelvac CircoFlex™ does not have serological changes, weaning number, weights of 7-week old pigs, number of marketing pigs, and death rate after the vaccine inoculation were recorded and effects were compared.

While the weaning numbers of groups A, B, and C were similar, the shipping numbers of the experimental groups A and B were greater than that of group C. In particular, the shipping number of group A according to the present invention was greater than that of group B (Ingelvac CircoFlex™) (Table 27). Furthermore, an average weight of the 7-week old pigs of groups A and B was greater than that of group C, and average weights of groups A and B were similar to each other (Table 27). Table 27 shows weaning number (rate), average weight of 7-week old pigs (n-20), and shipping number (rate).

Table 27

| Group | Weaning number (weaning rate[1]) | Shipping number (shipping rate [2]) | Average weight of pigs at the age of 7 weeks |
|---|---|---|---|
| A | 734(97.8) | 645 (86.0) | 15.9±0.9 |
| B | 95 (96.9) | 74 (75.5) | 15.0±1.1 |
| C | 47(95.9) | 25(51.0) | 14.3±1.7 |
| 1. Weaning rate = weaning number/number of animals x 100<br>2. Shipping rate = shipping number/number of animals x 100 | | | |

As described above, when the vaccine according to the present invention and a commercially available Ingelvac CircoFlex™ having similar effect were applied to farms for a predetermined period of time, the vaccine according to the present invention showed greater average weights of 7-week old pigs and shipping rates than Ingelvac CircoFlex™.

PCV2 GC0504 and PCV2 GC0505 according to according to an embodiment of the present invention has high replication ability in host cells.

The immunogenic composition according to an embodiment of the present invention has immunogenicity against at least one selected from the group consisting of various PCV2a and PCV2b.

According to the method of producing attenuated circovirus according to an embodiment of the present invention, attenuated circovirus may be produced.

According to a method of treating or preventing circovirus infection-associated diseases in animals, infection by at least one selected from the group consisting of PCV2a and PCV2b may be efficiently prevented or treated.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A composition for treating or preventing circovirus infection in animals, the composition comprising at least on attenuated virus selected from the group consisting of attenuated circovirus PCV2 GC0504 and PCV2 GC0505.

2. The composition of claim 1, wherein PCV2 GC0504 comprises a genome having a nucleotide sequence of SEQ ID NO: 1.

3. The composition of claim 1, wherein PCV2 GC0505 comprises a genome having a nucleotide sequence of SEQ ID NO: 4.

4. The composition of claim 1, wherein PCV2 GC0504 and PCV2 GC0505 are attenuated by the reaction with formaldehyde.

5. The composition of claim 1, wherein the amounts of PCV2 GC0504 and PCV2 GC0505 are respectively in the range of $10^{4.5}$ $TCID_{50}$ to $10^{6.5}$ $TCID_{50}$ at a single dose.

6. The composition of claim 1, wherein the circovirus used to prevent and treat circovirus infection is selected from the group consisting of PCV2a and PCV2b.

7. The composition of claim 1, wherein the circovirus used to prevent and treat circovirus infection comprises PCV2a and PCV2b.

8. The composition of claim 1, further comprising a veterinarily acceptable excipient.

9. The composition of claim 8, wherein the excipient is montanide gel, optionally,
in the range of 5 to 15% based on the total weight of the composition.

10. The composition of any one of claims 1 to 9, wherein the animal is selected from the group consisting of pig, mouse, and guinea pig.

11. The composition of any one of claims 1 to 10, wherein the composition is orally or parenterally administered, or is injected.

12. A method of producing attenuated circovirus, the method comprising reacting at least one virus selected from the group consisting of circovirus PCV2 GC0504 and PCV2 GC0505 with formalin to prepare attenuated circovirus, the formalin being preferably in the range of 0.2 to 0.5 wt% based on the total weight of the reaction solution.

13. Circovirus PCV2 GC0504 comprising a genome having a nucleotide sequence of SEQ ID NO: 1.

14. Circovirus PCV2 GC0505 comprising a genome having a nucleotide sequence of SEQ ID NO: 4.

FIG. 1

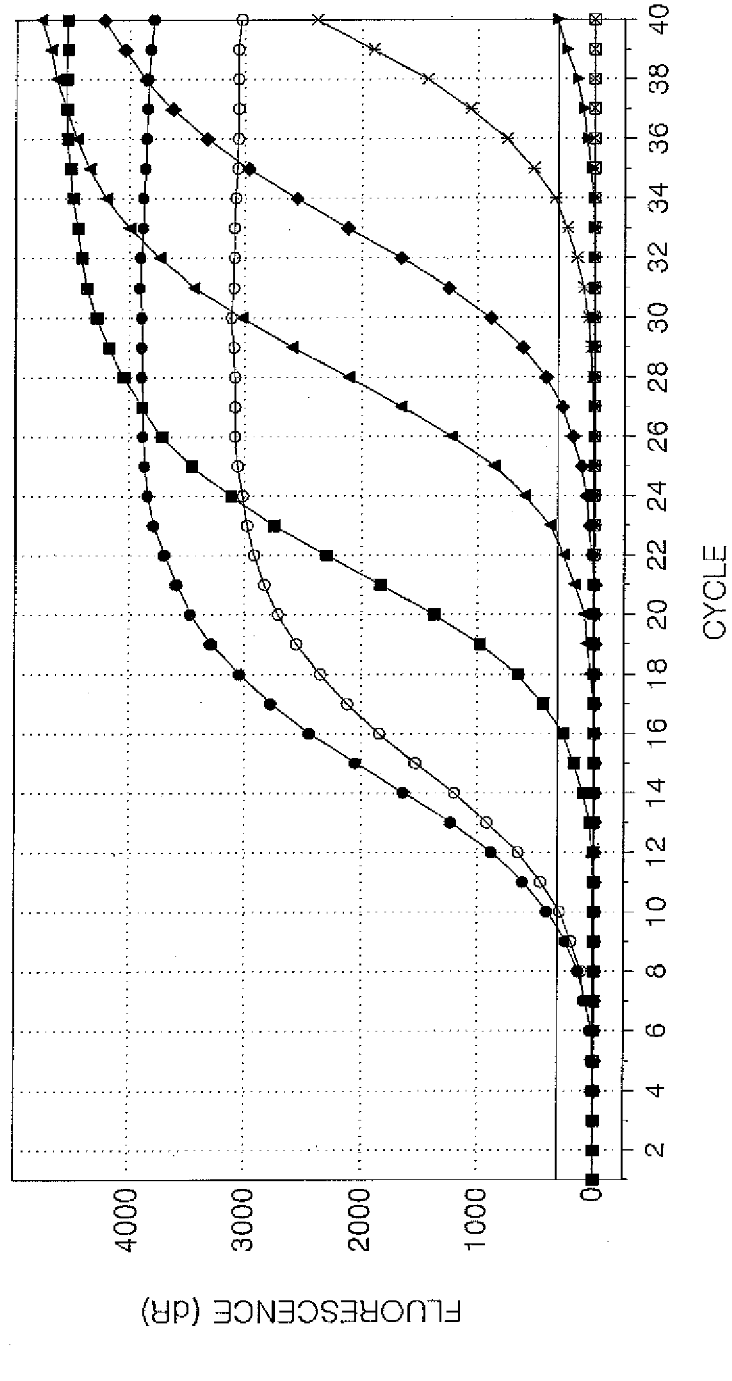
FLUORESCENCE (dR)
0
1000
2000
3000
4000
CYCLE
2 4 6 8 10 12 14 16 18 20 22 24 26 28 30 32 34 36 38 40

FIG. 2

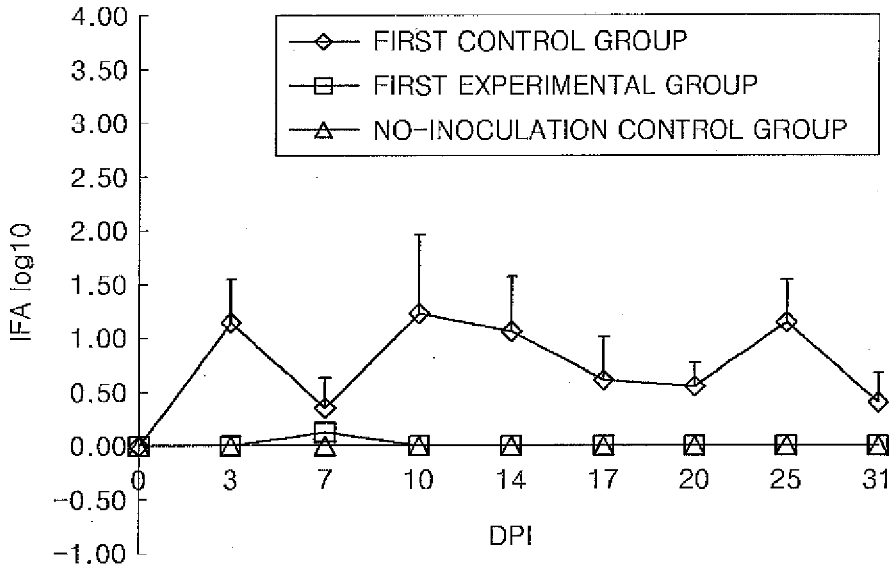

4.00
3.50
3.00
2.50
2.00
1.50
1.00
0.50
0.00
-0.50
-1.00
IFA log10
FIRST CONTROL GROUP
FIRST EXPERIMENTAL GROUP
NO-INOCULATION CONTROL GROUP
0
3
7
10
14
17
20
25
31
DPI

FIG. 3

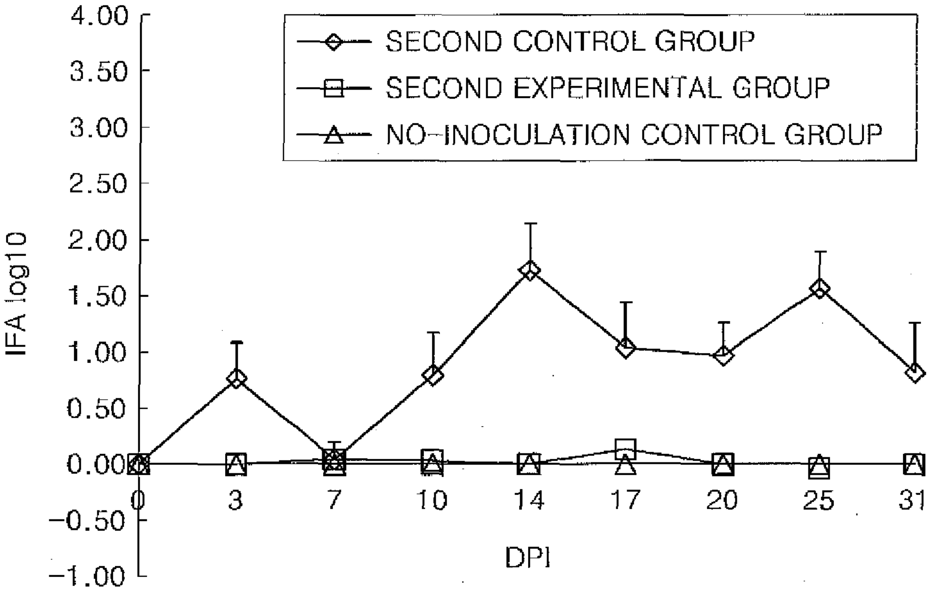

4.00
3.50
3.00
2.50
2.00
1.50
1.00
0.50
0.00
−0.50
−1.00
IFA log10
SECOND CONTROL GROUP
SECOND EXPERIMENTAL GROUP
NO-INOCULATION CONTROL GROUP
0
3
7
10
14
17
20
25
31
DPI

FIG. 4

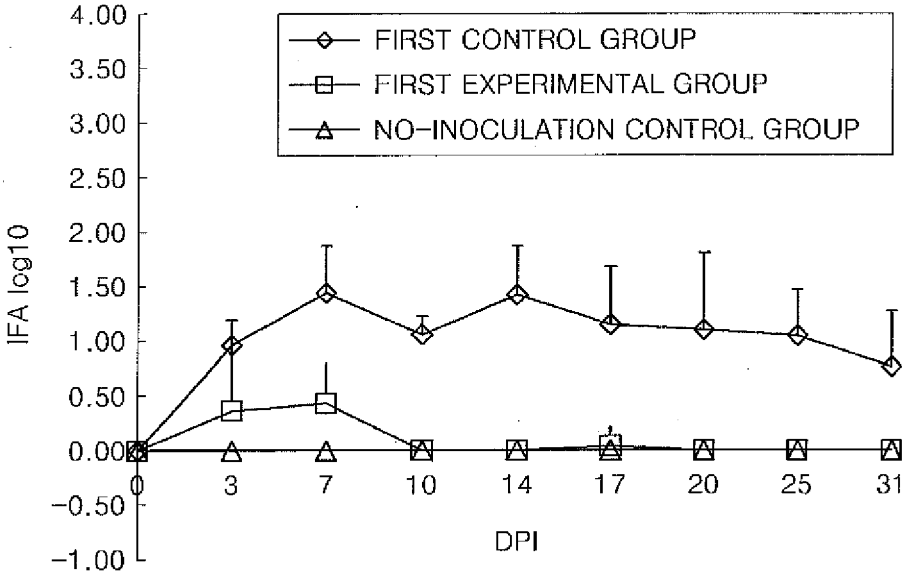
4.00
3.50
3.00
2.50
2.00
1.50
1.00
0.50
0.00
-0.50
-1.00
IFA log10
0
3
7
10
14
17
20
25
31
DPI
FIRST CONTROL GROUP
FIRST EXPERIMENTAL GROUP
NO-INOCULATION CONTROL GROUP

FIG. 5

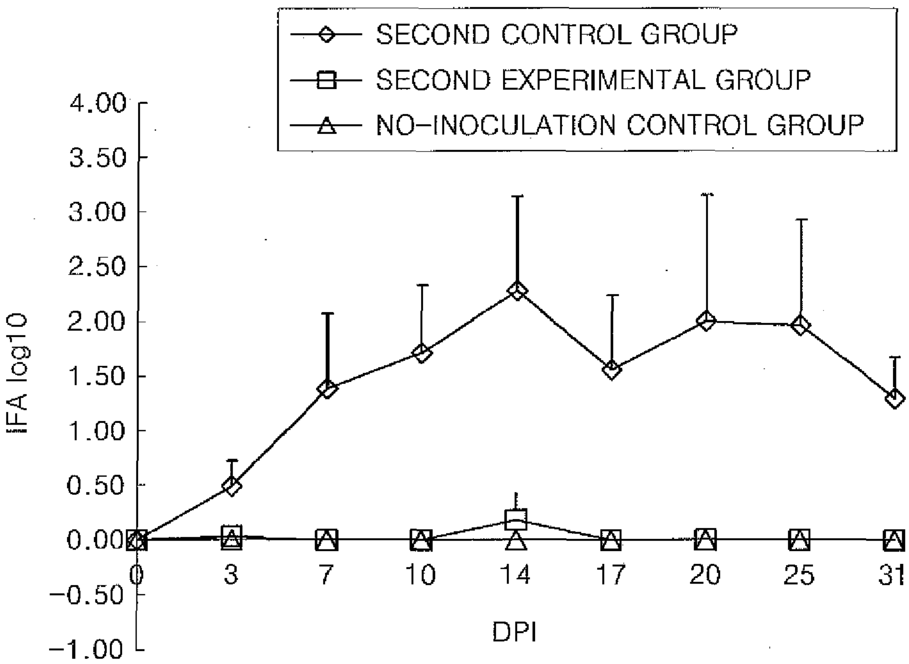
SECOND CONTROL GROUP
SECOND EXPERIMENTAL GROUP
NO-INOCULATION CONTROL GROUP
4.00
3.50
3.00
2.50
2.00
1.50
1.00
0.50
0.00
-0.50
-1.00
IFA log10
0
3
7
10
14
17
20
25
31
DPI

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020100010656
- US 7566562 B
- US 20040253270 A1


### Non-patent literature cited in the description


- **ALLAN et al.** *Arch Virol.,* 2000, vol. 145, 2421-2429
- **ALLAN et al.** *J Vet Med B.,* 2000, vol. 47, 81-94
- **FENAUX, M.** *J. Vir.,* January 2002, 541-551
- **LANGOHR.** *Vet pathol.,* 2010, vol. 47, 140-147
- **HANSEN.** *Vet Microbiol.,* 2010
- **FORT et al.** *Vet Microbiol,* 2007, vol. 125, 244-255

SEQUENCE LISTING

```
<110>  Green Cross Veterinary Products Co. Ltd.

<120>  Novel porcine circovirus type 2 and use thereof

<130>  SR 38073 (EP) / SG

<140>  EP 10XXXXXX.X
<141>  2010-05-21

<150>  KR 10-2010-0010656
<151>  2010-02-04

<160>  8

<170>  PatentIn version 3.5

<210>  1
<211>  1768
<212>  DNA
<213>  Porcine Circovirus type 2 GC0504

<220>
<221>  CDS
<222>  (51)..(992)
<223>  positive strand, replicase protein

<400>  1
accagcgcac ttcggcagcg gcagcacctc ggcagcacct cagcagcaac atg ccc         56
                                                       Met Pro
                                                       1

agc aag aag aat gga aga agc ggg ccc caa cca cat aaa agg tgg gtg       104
Ser Lys Lys Asn Gly Arg Ser Gly Pro Gln Pro His Lys Arg Trp Val
        5                   10                  15

ttc acg ctg aat aat cct tcc gaa gac gag cgc aag aaa ata cgg gag       152
Phe Thr Leu Asn Asn Pro Ser Glu Asp Glu Arg Lys Lys Ile Arg Glu
    20                  25                  30

ctc cca atc tcc ctg ttt gat tat ttt att gtt ggc gag gag ggt aat       200
Leu Pro Ile Ser Leu Phe Asp Tyr Phe Ile Val Gly Glu Glu Gly Asn
35                  40                  45                  50

gag gaa gga cga aca cct cac ctc cag ggg ttc gct aat ttt gtg aag       248
Glu Glu Gly Arg Thr Pro His Leu Gln Gly Phe Ala Asn Phe Val Lys
                55                  60                  65

aag caa act ttt aat aaa gtg aag tgg tat ttg ggt gcc cgc tgc cac       296
Lys Gln Thr Phe Asn Lys Val Lys Trp Tyr Leu Gly Ala Arg Cys His
            70                  75                  80

atc gag aaa gcc aaa gga act gat cag cag aat aaa gaa tat tgc agt       344
Ile Glu Lys Ala Lys Gly Thr Asp Gln Gln Asn Lys Glu Tyr Cys Ser
        85                  90                  95

aaa gaa ggc aac tta ctt att gaa tgt gga gct cct cga tct caa gga       392
Lys Glu Gly Asn Leu Leu Ile Glu Cys Gly Ala Pro Arg Ser Gln Gly
    100                 105                 110
```

```
caa cgg agt gac ctg tct act gct gtg agt acc ttg ttg gag agc ggg        440
Gln Arg Ser Asp Leu Ser Thr Ala Val Ser Thr Leu Leu Glu Ser Gly
115                 120                 125                 130

agt ctg gtg acc gtt gca gag cag cac cct gta acg ttt gtc aga aat        488
Ser Leu Val Thr Val Ala Glu Gln His Pro Val Thr Phe Val Arg Asn
                135                 140                 145

ttc cgc ggg ccg gct gaa ctt ttg aaa gtg agc ggg aaa atg cag aag        536
Phe Arg Gly Pro Ala Glu Leu Leu Lys Val Ser Gly Lys Met Gln Lys
            150                 155                 160

cgt gat tgg aag acc aat gta cac gtc att gtg ggg cca cct ggg tgt        584
Arg Asp Trp Lys Thr Asn Val His Val Ile Val Gly Pro Pro Gly Cys
        165                 170                 175

ggt aaa agc aaa tgg gct gct aat ttt gca gac ccg gaa acc aca tac        632
Gly Lys Ser Lys Trp Ala Ala Asn Phe Ala Asp Pro Glu Thr Thr Tyr
    180                 185                 190

tgg aaa cca cct aga aac aag tgg tgg gat ggt tac cat ggt gaa gaa        680
Trp Lys Pro Pro Arg Asn Lys Trp Trp Asp Gly Tyr His Gly Glu Glu
195                 200                 205                 210

gtg gtt gtt att gat gac ttt tat ggc tgg ctg ccg tgg gat gac cta        728
Val Val Val Ile Asp Asp Phe Tyr Gly Trp Leu Pro Trp Asp Asp Leu
                215                 220                 225

ctg aga ctg tgt gat cga tat cca ttg act gta gag act aaa ggt gga        776
Leu Arg Leu Cys Asp Arg Tyr Pro Leu Thr Val Glu Thr Lys Gly Gly
            230                 235                 240

act gta cct ttt ttg gcc cgc agt att ctg att acc agc aat cag acc        824
Thr Val Pro Phe Leu Ala Arg Ser Ile Leu Ile Thr Ser Asn Gln Thr
        245                 250                 255

ccg ttg gaa tgg tac tcc tca act gct gtc cca gct gta gaa gct ctc        872
Pro Leu Glu Trp Tyr Ser Ser Thr Ala Val Pro Ala Val Glu Ala Leu
    260                 265                 270

tat cgg agg att act tcc ttg gta ttt tgg aag aat gct aca gaa caa        920
Tyr Arg Arg Ile Thr Ser Leu Val Phe Trp Lys Asn Ala Thr Glu Gln
275                 280                 285                 290

tcc acg gag gaa ggg ggc cag ttc gtc acc ctt tcc ccc cca tgc cct        968
Ser Thr Glu Glu Gly Gly Gln Phe Val Thr Leu Ser Pro Pro Cys Pro
                295                 300                 305

gaa ttt cca tat gaa ata aat tac tgagtctttt ttatcacttc gtaatggttt      1022
Glu Phe Pro Tyr Glu Ile Asn Tyr
            310

ttattattca tttagggttt aagtgggggg tctttaagat taaattctct gaattgtaca     1082

tacatggtta cacggatatt gtagtcctgg tcgtatatac tgttttcgaa cgcagtgccg     1142

aggcctacgt ggtccacatt tctagaggtt tgtagcctca gccaaagctg attccttttg     1202

ttatttggtt ggaagtaatc aatagtggag tcaagaacag gtttgggtgt gaagtaacgg     1262
```

gagtggtagg agaagggttg ggggattgta tggcgggagg agtagtttac atatgggtca 1322

taggtttggg cattgacctt tggtacaaag ttatcatcta gaataacagc agtggagccc 1382

actcccctat caccctgggt gatgggggag cagggccagg attcaacctt aacctttctt 1442

attctgtagt attcagaggg tatagagatt ttgttggtcc cccctcccgg gggaacaaag 1502

tcgtcaagat taaatctcat catgtccacc gcccaggagg gcgttctgac tgtggtagcc 1562

ttgacagtat atccgaaggt gcgggagagg cgggtgttga agatgccatt tttccttctc 1622

caacggtagc ggtggcgggg gtggacgagc caggggcggc ggcggaggat ctggccaaga 1682

tggctgcggg ggcggtgtct tcttcttcgg taacgcctcc ttggatacgt catagctgaa 1742

aacgaaagaa gtgcgctgta agtatt 1768

<210> 2
<211> 314
<212> PRT
<213> Porcine Circovirus type 2 GC0504

<400> 2

```
Met Pro Ser Lys Lys Asn Gly Arg Ser Gly Pro Gln Pro His Lys Arg
1               5                   10                  15

Trp Val Phe Thr Leu Asn Asn Pro Ser Glu Asp Glu Arg Lys Lys Ile
            20                  25                  30

Arg Glu Leu Pro Ile Ser Leu Phe Asp Tyr Phe Ile Val Gly Glu Glu
        35                  40                  45

Gly Asn Glu Glu Gly Arg Thr Pro His Leu Gln Gly Phe Ala Asn Phe
    50                  55                  60

Val Lys Lys Gln Thr Phe Asn Lys Val Lys Trp Tyr Leu Gly Ala Arg
65                  70                  75                  80

Cys His Ile Glu Lys Ala Lys Gly Thr Asp Gln Gln Asn Lys Glu Tyr
                85                  90                  95

Cys Ser Lys Glu Gly Asn Leu Leu Ile Glu Cys Gly Ala Pro Arg Ser
            100                 105                 110

Gln Gly Gln Arg Ser Asp Leu Ser Thr Ala Val Ser Thr Leu Leu Glu
        115                 120                 125

Ser Gly Ser Leu Val Thr Val Ala Glu Gln His Pro Val Thr Phe Val
    130                 135                 140

Arg Asn Phe Arg Gly Pro Ala Glu Leu Leu Lys Val Ser Gly Lys Met
145                 150                 155                 160

Gln Lys Arg Asp Trp Lys Thr Asn Val His Val Ile Val Gly Pro Pro
                165                 170                 175
```

Gly Cys Gly Lys Ser Lys Trp Ala Ala Asn Phe Ala Asp Pro Glu Thr
180 185 190

Thr Tyr Trp Lys Pro Pro Arg Asn Lys Trp Trp Asp Gly Tyr His Gly
195 200 205

Glu Glu Val Val Val Ile Asp Asp Phe Tyr Gly Trp Leu Pro Trp Asp
210 215 220

Asp Leu Leu Arg Leu Cys Asp Arg Tyr Pro Leu Thr Val Glu Thr Lys
225 230 235 240

Gly Gly Thr Val Pro Phe Leu Ala Arg Ser Ile Leu Ile Thr Ser Asn
245 250 255

Gln Thr Pro Leu Glu Trp Tyr Ser Ser Thr Ala Val Pro Ala Val Glu
260 265 270

Ala Leu Tyr Arg Arg Ile Thr Ser Leu Val Phe Trp Lys Asn Ala Thr
275 280 285

Glu Gln Ser Thr Glu Glu Gly Gly Gln Phe Val Thr Leu Ser Pro Pro
290 295 300

Cys Pro Glu Phe Pro Tyr Glu Ile Asn Tyr
305 310

<210> 3
<211> 233
<212> PRT
<213> Porcine Circovirus type 2 GC0504

<220>
<221> PEPTIDE
<222> (1)..(233)
<223> PCV2 GC0504 ORF2

<400> 3

Met Thr Tyr Pro Arg Arg Arg Tyr Arg Arg Arg Arg His Arg Pro Arg
1 5 10 15

Ser His Leu Gly Gln Ile Leu Arg Arg Arg Pro Trp Leu Val His Pro
20 25 30

Arg His Arg Tyr Arg Trp Arg Arg Lys Asn Gly Ile Phe Asn Thr Arg
35 40 45

Leu Ser Arg Thr Phe Gly Tyr Thr Val Lys Ala Thr Thr Val Arg Thr
50 55 60

Pro Ser Trp Ala Val Asp Met Met Arg Phe Asn Leu Asp Asp Phe Val
65 70 75 80

Pro Pro Gly Gly Gly Thr Asn Lys Ile Ser Ile Pro Ser Glu Tyr Tyr
85 90 95

Arg Ile Arg Lys Val Lys Val Glu Ser Trp Pro Cys Ser Pro Ile Thr
100 105 110

```
Gln Gly Asp Arg Gly Val Gly Ser Thr Ala Val Ile Leu Asp Asp Asn
        115                 120                 125

Phe Val Pro Lys Val Asn Ala Gln Thr Tyr Asp Pro Tyr Val Asn Tyr
    130                 135                 140

Ser Ser Arg His Thr Ile Pro Gln Pro Phe Ser Tyr His Ser Arg Tyr
145                 150                 155                 160

Phe Thr Pro Lys Pro Val Leu Asp Ser Thr Ile Asp Tyr Phe Gln Pro
                165                 170                 175

Asn Asn Lys Arg Asn Gln Leu Trp Leu Arg Leu Gln Thr Ser Arg Asn
            180                 185                 190

Val Asp His Val Gly Leu Gly Thr Ala Phe Glu Asn Ser Ile Tyr Asp
        195                 200                 205

Gln Asp Tyr Asn Ile Arg Val Thr Met Tyr Val Gln Phe Arg Glu Phe
    210                 215                 220

Asn Leu Lys Asp Pro Pro Leu Lys Pro
225                 230


<210>  4
<211>  1767
<212>  DNA
<213>  Porcine Circovirus type 2 GC0505

<220>
<221>  CDS
<222>  (51)..(992)
<223>  positive strand, replicase protein

<400>  4
accagcgcac ttcggcagcg gcagcacctc ggcagcacct cagcagcaac atg ccc        56
                                                       Met Pro
                                                       1

agc aag aag aat gga aga agc gga ccc caa ccc cat aaa agg tgg gtg      104
Ser Lys Lys Asn Gly Arg Ser Gly Pro Gln Pro His Lys Arg Trp Val
        5                   10                  15

ttc act ctg aat aat cct tcc gaa gac gag cgc aag aaa ata cgg gat      152
Phe Thr Leu Asn Asn Pro Ser Glu Asp Glu Arg Lys Lys Ile Arg Asp
    20                  25                  30

ctt cca ata tcc ctg ttt gat tat ttt att gtt ggc gag gag ggt aat      200
Leu Pro Ile Ser Leu Phe Asp Tyr Phe Ile Val Gly Glu Glu Gly Asn
35                  40                  45                  50

gag gaa gga cga aca cct cac ctc cag ggg ttc gct aat ttt gtg aag      248
Glu Glu Gly Arg Thr Pro His Leu Gln Gly Phe Ala Asn Phe Val Lys
                55                  60                  65

aag cag act ttt aat aaa gtg aag tgg tat ttg ggt gcc cgc tgc cac      296
Lys Gln Thr Phe Asn Lys Val Lys Trp Tyr Leu Gly Ala Arg Cys His
            70                  75                  80
```

```
atc gag aaa gcg aaa gga aca gat cag cag aat aaa gaa tac tgc agt        344
Ile Glu Lys Ala Lys Gly Thr Asp Gln Gln Asn Lys Glu Tyr Cys Ser
        85                  90                  95

aaa gaa ggc aac tta ctg atg gag tgt gga gct cct aga tct cag gga        392
Lys Glu Gly Asn Leu Leu Met Glu Cys Gly Ala Pro Arg Ser Gln Gly
    100                 105                 110

caa cgg agt gac ctg tct act gct gtg agt acc ttg ttg gag agc ggg        440
Gln Arg Ser Asp Leu Ser Thr Ala Val Ser Thr Leu Leu Glu Ser Gly
115                 120                 125                 130

agt ctg gtg acc gtt gca gag cag cac cct gta acg ttt gtc aga aat        488
Ser Leu Val Thr Val Ala Glu Gln His Pro Val Thr Phe Val Arg Asn
                135                 140                 145

ttc cgc ggg ctg gct gaa ctt ttg aaa gtg agc ggg aaa atg cag aag        536
Phe Arg Gly Leu Ala Glu Leu Leu Lys Val Ser Gly Lys Met Gln Lys
            150                 155                 160

cgt gat tgg aag act aat gta cac gtc att gtg ggg cca cct ggg tgt        584
Arg Asp Trp Lys Thr Asn Val His Val Ile Val Gly Pro Pro Gly Cys
        165                 170                 175

ggt aaa agc aaa tgg gct gct aat ttt gca gac ccg gaa acc aca tac        632
Gly Lys Ser Lys Trp Ala Ala Asn Phe Ala Asp Pro Glu Thr Thr Tyr
    180                 185                 190

tgg aaa cca cct aga aac aag tgg tgg gat ggt tac cat ggt gaa gaa        680
Trp Lys Pro Pro Arg Asn Lys Trp Trp Asp Gly Tyr His Gly Glu Glu
195                 200                 205                 210

gtg gtt gtt att gat gac ttt tat ggc tgg ctg ccc tgg gat gat cta        728
Val Val Val Ile Asp Asp Phe Tyr Gly Trp Leu Pro Trp Asp Asp Leu
                215                 220                 225

ctg aga ctg tgt gat cga tat cca ttg act gta gag act aaa ggt gga        776
Leu Arg Leu Cys Asp Arg Tyr Pro Leu Thr Val Glu Thr Lys Gly Gly
            230                 235                 240

act gta cct ttt ttg gcc cgc agt att ctg att acc agc aat cag acc        824
Thr Val Pro Phe Leu Ala Arg Ser Ile Leu Ile Thr Ser Asn Gln Thr
        245                 250                 255

ccg ttg gaa tgg tac tcc tca act gct gtc cca gct gta gaa gct ctt        872
Pro Leu Glu Trp Tyr Ser Ser Thr Ala Val Pro Ala Val Glu Ala Leu
    260                 265                 270

tat cgg agg att act tcc ttg gta ttt tgg aag aat gct aca gaa caa        920
Tyr Arg Arg Ile Thr Ser Leu Val Phe Trp Lys Asn Ala Thr Glu Gln
275                 280                 285                 290

tcc acg gag gaa ggg ggc cag ttc gtc acc ctt tcc ccc cca tgc cct        968
Ser Thr Glu Glu Gly Gly Gln Phe Val Thr Leu Ser Pro Pro Cys Pro
                295                 300                 305

gaa ttt cca tat gaa ata aat tac tgagtctttt ttatcacttc gtaatggttt      1022
Glu Phe Pro Tyr Glu Ile Asn Tyr
            310
```

```
ttattattca ttaagggtta agtgggggt ctttaagatt aaattctctg aattgtacat     1082
acatggttac acggatattg tattcctggt cgtatatact gttttcgaac gcagtgccga    1142
ggcctacgtg gtctacattt ccagtagttt gtagtctcag ccacagctga tttcttttgt    1202
tgtttggttg gaagtaatca atagtggaat ctaggacagg tttgggggta aagtagcggg    1262
agtggtagga gaagggctgg gttatggtat ggcgggagga gtagtttaca taggggtcat    1322
aggtgagggc tgtggccttt gttacaaagt tatcatctag aataacagca ctggagccca    1382
ctcccctgtc accgtgggtg atcggggagc agggccagaa ttcaacctta acctttctta    1442
ttctgtagta cccatagggc acagagcggg ggtttgagcc ccctcctggg ggaagaaagt    1502
cattaatatt gaatctcatc atgtccaccg cccaggaggg cgttttgact gtggttcgct    1562
tgatagtata tccgaaggtg cgggagaggc gggtgttgaa gatgccattt ttccttctcc    1622
agcggtaacg gtggcggggg tggacgagcc aggggcggcg gcggaggatc tggccaagat    1682
ggctgcgggg gcggtgtctt cttctccggt aacgcctcct tggatacgtc atatctgaaa    1742
acgaaagaag tgcgctgtaa gtatt                                          1767
```

<210> 5
<211> 314
<212> PRT
<213> Porcine Circovirus type 2 GC0505

<400> 5

```
Met Pro Ser Lys Lys Asn Gly Arg Ser Gly Pro Gln Pro His Lys Arg
1               5                   10                  15

Trp Val Phe Thr Leu Asn Asn Pro Ser Glu Asp Glu Arg Lys Lys Ile
            20                  25                  30

Arg Asp Leu Pro Ile Ser Leu Phe Asp Tyr Phe Ile Val Gly Glu Glu
        35                  40                  45

Gly Asn Glu Glu Gly Arg Thr Pro His Leu Gln Gly Phe Ala Asn Phe
    50                  55                  60

Val Lys Lys Gln Thr Phe Asn Lys Val Lys Trp Tyr Leu Gly Ala Arg
65                  70                  75                  80

Cys His Ile Glu Lys Ala Lys Gly Thr Asp Gln Gln Asn Lys Glu Tyr
                85                  90                  95

Cys Ser Lys Glu Gly Asn Leu Leu Met Glu Cys Gly Ala Pro Arg Ser
            100                 105                 110

Gln Gly Gln Arg Ser Asp Leu Ser Thr Ala Val Ser Thr Leu Leu Glu
        115                 120                 125

Ser Gly Ser Leu Val Thr Val Ala Glu Gln His Pro Val Thr Phe Val
```

```
    130                 135                 140

Arg Asn Phe Arg Gly Leu Ala Glu Leu Leu Lys Val Ser Gly Lys Met
145                 150                 155                 160

Gln Lys Arg Asp Trp Lys Thr Asn Val His Val Ile Val Gly Pro Pro
                165                 170                 175

Gly Cys Gly Lys Ser Lys Trp Ala Ala Asn Phe Ala Asp Pro Glu Thr
            180                 185                 190

Thr Tyr Trp Lys Pro Pro Arg Asn Lys Trp Trp Asp Gly Tyr His Gly
        195                 200                 205

Glu Glu Val Val Val Ile Asp Asp Phe Tyr Gly Trp Leu Pro Trp Asp
    210                 215                 220

Asp Leu Leu Arg Leu Cys Asp Arg Tyr Pro Leu Thr Val Glu Thr Lys
225                 230                 235                 240

Gly Gly Thr Val Pro Phe Leu Ala Arg Ser Ile Leu Ile Thr Ser Asn
                245                 250                 255

Gln Thr Pro Leu Glu Trp Tyr Ser Ser Thr Ala Val Pro Ala Val Glu
            260                 265                 270

Ala Leu Tyr Arg Arg Ile Thr Ser Leu Val Phe Trp Lys Asn Ala Thr
        275                 280                 285

Glu Gln Ser Thr Glu Glu Gly Gly Gln Phe Val Thr Leu Ser Pro Pro
    290                 295                 300

Cys Pro Glu Phe Pro Tyr Glu Ile Asn Tyr
305                 310


<210>  6
<211>  233
<212>  PRT
<213>  Porcine Circovirus type 2 GC0505

<220>
<221>  PEPTIDE
<222>  (1)..(233)
<223>  PCV2 GC0505 ORF2

<400>  6

Met Thr Tyr Pro Arg Arg Arg Tyr Arg Arg Arg Arg His Arg Pro Arg
1               5                   10                  15

Ser His Leu Gly Gln Ile Leu Arg Arg Arg Pro Trp Leu Val His Pro
            20                  25                  30

Arg His Arg Tyr Arg Trp Arg Arg Lys Asn Gly Ile Phe Asn Thr Arg
        35                  40                  45

Leu Ser Arg Thr Phe Gly Tyr Thr Ile Lys Arg Thr Thr Val Lys Thr
    50                  55                  60
```

```
Pro Ser Trp Ala Val Asp Met Met Arg Phe Asn Ile Asn Asp Phe Leu
65                  70                  75                  80

Pro Pro Gly Gly Gly Ser Asn Pro Arg Ser Val Pro Tyr Gly Tyr Tyr
                85                  90                  95

Arg Ile Arg Lys Val Lys Val Glu Phe Trp Pro Cys Ser Pro Ile Thr
            100                 105                 110

His Gly Asp Arg Gly Val Gly Ser Ser Ala Val Ile Leu Asp Asp Asn
        115                 120                 125

Phe Val Thr Lys Ala Thr Ala Leu Thr Tyr Asp Pro Tyr Val Asn Tyr
    130                 135                 140

Ser Ser Arg His Thr Ile Thr Gln Pro Phe Ser Tyr His Ser Arg Tyr
145                 150                 155                 160

Phe Thr Pro Lys Pro Val Leu Asp Ser Thr Ile Asp Tyr Phe Gln Pro
                165                 170                 175

Asn Asn Lys Arg Asn Gln Leu Trp Leu Arg Leu Gln Thr Thr Gly Asn
            180                 185                 190

Val Asp His Val Gly Leu Gly Thr Ala Phe Glu Asn Ser Ile Tyr Asp
        195                 200                 205

Gln Glu Tyr Asn Ile Arg Val Thr Met Tyr Val Gln Phe Arg Glu Phe
    210                 215                 220

Asn Leu Lys Asp Pro Pro Leu Asn Pro
225                 230


<210>  7
<211>  20
<212>  DNA
<213>  Artificial Sequence

<220>
<223>  Forward primer

<400>  7
taggttaggg ctgtggcctt                                                    20


<210>  8
<211>  20
<212>  DNA
<213>  Artificial Sequence

<220>
<223>  Reverse primer

<400>  8
ccgcaccttc ggatatactg                                                    20
```